# EUROPEAN PATENT APPLICATION

(11) **EP 2 014 659 A1**
(43) Date of publication of application: **14.01.2009**
(21) Application number: 07011179.4
(22) Date of filing: 07.06.2007
(51) Int. Cl.: C07D 471/04, C07D 473/40, C07H 21/00, C09K 9/02

(54) **New hybrid compounds of nucleobases and organic redox molecules and their use**

(71) Applicant: Universität Osnabrück, 49069 Osnabrück (DE)
(72) Inventor: Asaftei, Simona Carmen, 49078, Osnabrück (DE)
(74) Representative: Kröncke, Rolf

(57) **Abstract**

The present invention relates to new compounds as well as methods for the synthesis of the same, said compounds comprise a redox active group, e.g. a 4,4-bipyridine (viologen), a 1,2-di(4-pyridyl)ethane, a 2,7-diazapyrene or 2,9-diazaperylene group and a N-heterocyclic nucleobase (HB), also referred to as base.

## Description

The present invention relates to new compounds as well as methods for the synthesis of the same. Said compounds comprise a redox active group, e.g. a 4,4'-bipyridine (viologen), a 1,2'-di(4-pyridyl)ethane, a 2,7-diazapyrene or a 2,9-diazaperylene group and an N-heterocyclic nucleobase (HB), also referred to as base.

Said nucleobase group and said redox active group are linked via a spacer moiety linking the nucleobase group with the redox active group. Particularly preferred are viologen derivatives as redox active group. In addition, the present invention relates to methods for the production of said hybrid compounds. Furthermore, the present invention relates to an electrochromic device like an electrode using the electrochromic compounds as an electrochromic material. Finally, the present invention concerns nucleic acid molecules wherein the hybrid compounds having the nucleobase moiety are present in form of derivatives of nucleotides or nucleosides.

### Background art

Nucleobases are known to represent parts of RNA and DNA. These nucleobases include cytosine, guanine, adenine, thymine, uracil, xanthine, and hypoxanthine. Adenine, guanine, inosine, xanthine and hypoxanthine as well as modified derivatives thereof belong to the double ring class of molecules called purines, cytosine, thymine and uracil belong to the class of pyrimidines. As integral part of nucleic acids said bases have a central role, for example in carrying the genetic information. Modified bases, such as aza- and deazapurines are well known derivatives of the nucleobases occurring in nature and display various chemical and biological properties. In this connection, reference is made to the publication of Rosemeyer, H., Chemistry and Biodiversity 2004, 1, 361-401, Lagoja, I. M., Chemistry and Biodiversity 2005, 2, 1-50 and Grierson, D. S., Bioorg. Med. Chem., 2006, 14, 3987-4006. Nucleobases as a part of the nucleotides forming DNA, RNA or other nucleic acid molecules represent particularly useful entities in nucleic acid chemistry and biology. For example, in the field of diagnostics, nucleic acid molecules are widely used as detecting units, being labelled with radioactive isotopes or fluorescent moieties.

Electrochromic materials which change their optical properties in response to an electric field and which can be returned to their original state applying a field reverse are divided at least into five major families:
- metal oxide systems
- Prussian blue systems
- conducting polymers
- metallopolymers and phthalocyanines
- bipyridinium salts, in particular viologens.

These materials have major advantages, such as a small switching voltage, a high contrast between their coloured states, high colour purity in those states, and reversible colouring or bleaching.

Various electrochromic compounds are described in the art. For example, WO 2004/067673 discloses electrochromic compounds based on viologens. Viologens are diquaternary derivatives of 4,4'-bipyridyl. The name comes from the fact, that this class of compounds is easily reduced to the radical monocation, which is intensively blue coloured. Further reduction results in a yellowish compound. Diquaternary derivatives of 2,2'-bipyridine give a green radical anion.

The various application areas of viologens are described for example in Monk, P.M.S., John Wiley and Sons, Qichester, 1998. One of the most prominent representatives of the family of viologens is the so-called Paraquat (1,'1'-Dimetyl-4,4'-bipyridinium dichloride), a potent contact herbicide.

The three oxidation states of 4,4'-bipyridinium are shown below:

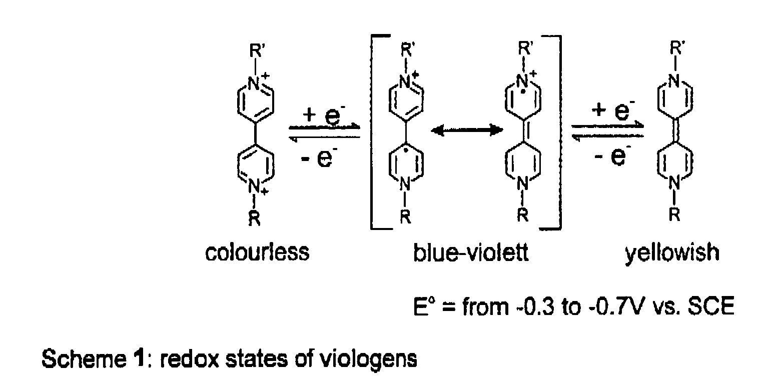

Due to the three different oxidation states of the 1,1'-disubstituted 4,4'-bipyridinium compounds and the high reversibility of the redox states, the viologens are of particular interest. The most stable form of the viologen is the colourless dication state on left side of the above scheme (Mortimer, R.J., Chemical Society, Reviews, 1997, 26(3), 147-156).

Reductive electron transfer to the dication results in the formation of blue to violet radical cations (one-electron transfer). A double electron transfer to the dication results in yellowish compounds. As mentioned before, using viologens allows the production of stable electrochromic high resolution pictures, see for example Ph.D. Thesis, Asaftei, C.S., Universität Osnabrück, July 2005.

The stability of said different forms of viologens is attributable to the delocalization of the radical electron throughout the Π-frame work of the bipyridine nucleus, the R and R'-substituents commonly bearing some of the charge.

As mentioned before, viologens are described for example in WO 2004/067673 relating to viologen compounds composed of a 4,4'-dipyridinium unit being substituted with a phosphonate moiety containing a group at position 1 and with the same or a different group at the 1'-position which do not cover the whole range of visible light. The compounds disclosed in WO 2004/067673 are benzyl, naphtyl or phenyl derivatives at the 1'-position on the bipyridinium derivatives. The compounds described therein display a green or blue to black colour only in the reduced state.

At present, no viologen derivatives or other electrochromic material have been described having e.g. an orange or red colour in the reduced state.

Furthermore, until today no compounds have been described combining the properties of nucleobases or derivatives thereof and electrochromic components.

### Summary of the present invention

The present invention relates to new hybrid molecules characterized in having redox active group(s) or moiety(ies) covalently linked to N-heterocyciic nucleobase moiety(ies) via spacer moiety(ies) which link the nucleobase moiety(ies) with the redox active moiety(ies).

The present invention further relates to intermediate products of said hybrid molecules as well as to the production of said hybrid molecules comprising an N-heterocyclic nucleobase and a redox active moiety which are linked via a spacer moiety as well as intermediates thereof. These hybrid moieties which are also referred to as hybrid units may be present as an autonomous unit or may be part of molecules, in particular of dendritic units, like dendrimers, connected via connector groups to core molecules.

Furthermore, the present invention relates to a method for the production of hybrid compounds according to the present invention having a moiety of an N-heterocyclic base or derivatives thereof, in particular, a purine or pyrimidine nucleobase or derivatives thereof. Said method comprises the step of reacting said N-heterocyclic nucleobase such as a purine or pyrimidine nucleobase or derivatives thereof with a spacer moiety. Said spacer has at least two reactive groups whereby reaction of the spacer with the nucleobase or a derivative thereof gives an intermediate compound consisting of said nucleobase and the spacer moiety with a remaining reactive group. Said spacer moiety is bound to an N- or C-atom present in the N-heterocyclic nucleobase, such as the purine or pyrimidine nucleobase or derivatives thereof.

In a further embodiment, the present invention relates to an electrochromic material, comprising the hybrid compounds or molecules comprising hybrid units according to the present invention as well as polymers comprising said hybrid units.

In addition, the present invention concerns an electrochromic device comprising the electrochromic material according to the present invention like an electrode.

Furthermore, the present invention provides nucleotide or nucleoside derivatives composed of the hybrid molecules and a sugar residue which may be phosphorylated. These nucleotides can be part of nucleic acids claimed herein; said nucleic acid molecules are particularly useful into termination of nucleic acids, e.g. in diagnostics or alone or as parts of nucleic acids for medical and pharmaceutical applications.

### Brief description of the figures

- Figure 1:: Figure 1 shows the results obtained after ion exchange HPLC of the oligomer 5'-D(TTTGGGGTTTT)-3' in the presence of a compound according to the present invention, namely 1-ethyl-4-pyridine-4-pyrridinium bromide as described in experiment 1.
- Figure 2:: Figure 2 shows the Vis spectra of a solution of a compound of the present invention, namely compound 7 as shown in scheme 16, in the reduction state. The measurement was started at 0 V and was continued to -0.600 V. The absorption maxima increased when decreasing the potential to about -0.500 V.
- Figure 3:: Figure 3 provides data obtained after cyclic voltammetry of the compound 7, see scheme 16, on a TIO₂ electrode as described in experiment 4.
- Figure 4:: Figure 4 provides the cyclic voltammetry data obtained for compound 6 shown in scheme 1, namely 7-(2-bromoethyl)-4-chloro-7H-pyrrolo[2,3-di]pyrimidine measured with glassy carbon electrode in 1MTBABr/H₂O v= 20 mV.
- Figure 5:: Figure 5 provides the Vis spectra of compound 7/TIO₂-electrode, Compound 7 is shown e.g. in scheme 16, in the reduction state in H₂O/TBABr 1 M as function of the electrode potential -0.50 to -0.9 V versus Ag/AgCl. The insert of figure 5 provides the absorbance of said electrode at 570 nm versus electrode potential -0.50 to -0.60 V. Details of the experiment are outlined in experiment 4.
- Figure 6:: Figure 6 shows modified TiO₂ electrodes with a viologen dendrimer 0^{th} generation with 3 different end groups: a) ethyl-; b) phenyl-; c) 4-chloropyrrolo [2,3-di] pyrimidine (compound 7 in scheme 16) in the periphery (in three electrode arrangements: a) and b) MeCn-NeClO4 1 Mol, c) H₂O/TBABr 1 Mol versus AgCl. Shown is the effect of a heterocyclic base as an end group and in a dendrimer of the 0^{th} generation with viologen as redox units as well as the coloration of said electrodes compared with well known viologen dendrimers.

### Detailed description of the invention

The present invention relates to hybrid compounds having a nucleobase or derivatives thereof, a redox active moiety and a spacer moiety linking the nucleobase moiety with the redox active moiety.

As used herein, the term "nucleobase moiety" or "base moiety" refers to a part or component of the claimed hybrid compound which is an N-heterocyclic group. Typically, the nucleobases are N-heterocyclic groups present in naturally-occurring nucleosides or nucleotides, e.g. purine- or pyrimidine bases as well as isosteres thereof, preferably aza- and deazapurines or -pyrimidines. Said nucleobase moieties may be substituted. In this connection, the term "which may be substituted" means the presence of at least one substituent of a C1 to C4 alkyl, C1 to C4 alkoxy, which may be a straight chain or branched and may preferably be methyl, trifluoromethyl, cyano, and nitro, halogen, like fluorine, chlorine, bromine or iodine.

The moiety having redox activity as well as the nucleobase moiety may be optionally substituted by one or more of the above mentioned substituents which may be identical or different.

As used herein, the term "redox active moiety" or "moiety having redox activity" refers to a moiety being part of the claimed hybrid compound having a Π-conjugated system. In particular, the redox active group refers to groups or moieties consisting of 4,4'-dipyridine, 2,2'-dipyridine, 1,2-di(4-pyridine)ethane, 2,7-diazapyrene and 2,9-diazaperylene as well as derivatives thereof which may be substituted at least once with the substituents mentioned above.

As used herein, the term "electrochromic" refers to the property of compounds or materials containing said compounds undergoing a colour change when voltage is applied once, and the coloured change is persistent.

Hybrid compounds described herein display new chemical, physical and biological properties based on the properties of each of the moieties of said hybrid compounds. In addition, new none-colligative properties are provided. In the following each of the moieties are described in detail.

### Hybrid compounds

The following scheme shows the structure of the hybrid molecules according to the present invention which may represent hybrid units of higher molecules like dendrimers. In the following, said structures may also refer to hybrid compound branches. Said structures are composed of an N-heterocyclic base moiety, HB, or a special head group, SHG, containing an N-heterocyclic nucleobase, a spacer moiety, S, and the redox active group, RG, see scheme 2.

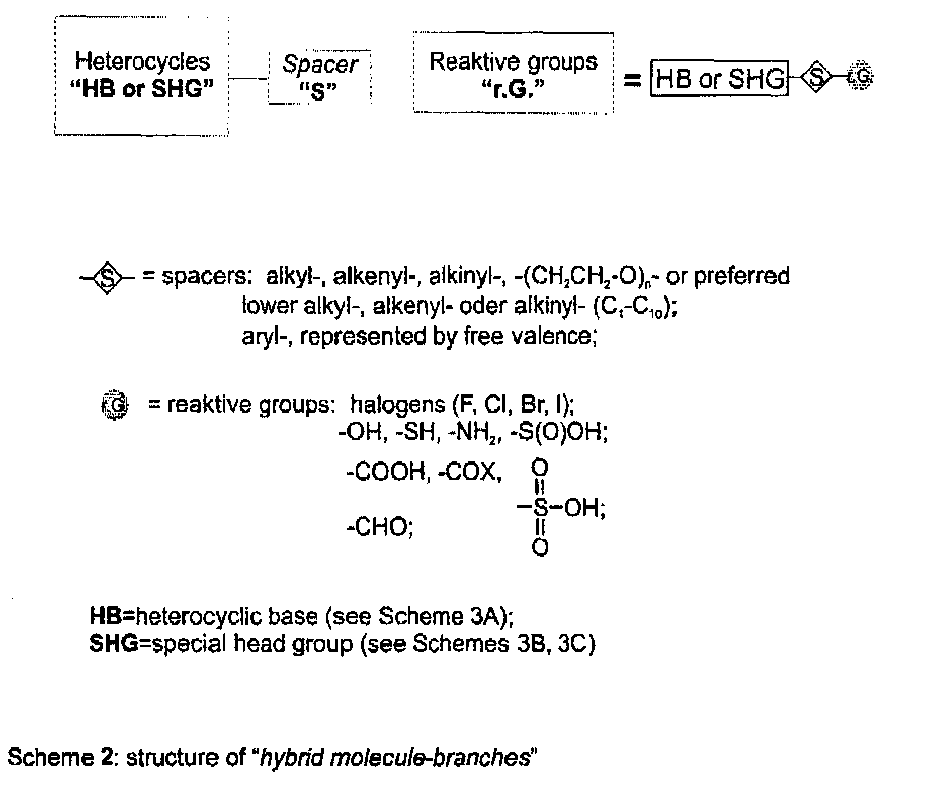

### N-Heterocyclic nucleobases, HB

As indicated above, the base moiety of the present invention is an N-heterocyclic base moiety, preferably a purine or pyrimidine nucleobase or a derivative thereof. Another preferred embodiment of the nucleobase comprises aza- and deaza derivatives of said purine and pyrimidine bases. Purine nucleobases include adenine, guanine, xanthine, and hypoxanthine while pyrimidine nucleobases comprise uracil, thymine and cytosine. Said nucleobases may also be natural-occurring modified nucleobases such as 5-methylcytosine, pseudouracil or 7-methylguanine.

Particular embodiments of the N-heterocyclic base moieties are shown in the scheme below, namely, N-heterocyclic bases of the purine and pyrimidine class as well as aza- and deaza- derivatives thereof. That is, various embodiments of nucleobase moieties according to the present invention are shown which, when linked via a spacer molecule having a terminal reactive group, may be linked to a redox active moiety as defined herein.

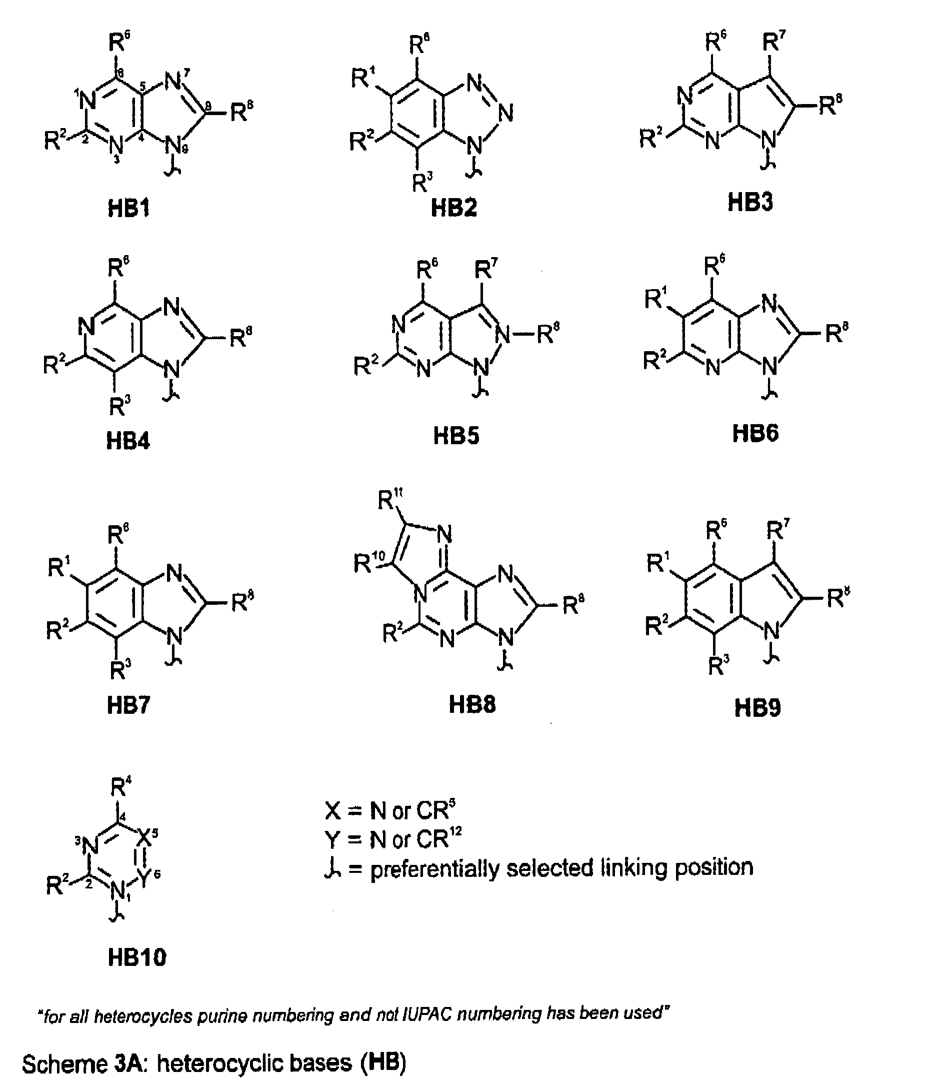

The preparations of the N-heterocyclic compounds shown in scheme 3A are known to the skilled person and most of said compounds are commercially available. Furthermore, reference is made to the following publication describing the synthese s of the above mentioned heterocyclic bases: F. Seela, N. Ramzaeva, H. Rosemeyer in: "Science of Synthesis, Product Class 17, 2003, Vol. 16, pp 945-1108; J. Davoll, J. Chem. Soc. 1960, 131, Robins, J. Am. Chem. Soc. 1956, 78, 784; F. Seela, M. Zulauf, G. Becher, Nucleosides, Nucleotides 1997, 16, 305.

In scheme 3a above, substituents R¹ - R¹² are independently chosen from a group consisting of H, halogens, -OR¹³, -SR¹⁴, -(C₁-C₁₀) alkyl, -(C₂-C₁₀)-alkenyl, -(C₂-C₁₀)-alkynyl. -NO₂, -NR¹⁵R¹⁶, -COO(C₁-C₄) alkyl, with the proviso that R¹³, R¹⁴, R¹⁵, R¹⁶ and R¹⁷ are independently chosen from a group consisting of -H, -(C₁-C₁₀)-alkyl, - (C₂-C₁₀)-alkenyl, -(C₂-C₁₀)-alkynyl, -(C₂-C₂₂)-aryl, or heteroaryl-, a protecting group or a reporter group.

A protecting group is a chemical group that is attached to a functional moiety (for example to the oxygen in a hydroxyl group, replacing the hydrogen) to protect the functional group from reacting in an undesired way. A protecting group is further defined by the fact that it can be removed without destroying the biological activity of the molecule formed, here the binding of the nucleic acid binding compound to a nucleic acid. Suitable protecting groups are known to a man skilled in the art.

Especially preferred protecting groups for example for hydroxyl groups are from the trityl group, for example dimethoxytrityl.

Preferred protecting groups at exocyclic amino groups in formula I are the acyl groups, most preferred the benzoyl (Bz), phenoxyacetyl (Pac) or acetyl or formyl, and the N,N-dialkylformamidine group, preferentially the dimethyl-, diisobutyl-, and the din-butylformamidine group.

Preferred O-protecting groups in OR¹³ are the aroyl, the acyl and the silyl groups. Among these most preferred is the benzoyl group. Preferred silyl groups are the trialkylsilyl groups such as triethylsilyl and triisopropylsilyloxymethyl (TOM) [X. Wu, S. Pitsch, Nucleich Acids Res. 1998, 26, 4315].

Reporter groups are generally groups that make the hybrid compound distinguishable from the remainder of the liquid. This distinction can be either effected by selecting the reporter group from the group of directly or indirectly detectable groups or from the groups of immobilized or immobilizable groups.

Directly detectable groups are for example fluorescent compounds, like fluorescein or its derivatives such as hexachlorofluorescein and hexafluorofluorescein, rhodamines, BODIPY dyes, psoralenes, squaraines, porphyrines, fluorescent particles, bioluminescent compounds, like acridinium esters and luminal, or the cyanine dyes, like CY-5. Examples of such compounds are disclosed in EP 0 680 969. Further spin labels like TEMPO, electrochemical detectable groups, ferrocene, viologene, heavy metal chelates and electro-chemiluminiscent labels, like ruthenium bispyridyl complexes, and naphtochinones, quencher dyes, like dabcyl and nuclease-active complexes, for example of Fe and Cu, are useful detectable groups. Indirectly detectable groups are groups than can be recognized by another moiety which is directly or indirectly labelled. Examples of such indirect detectable groups are for example haptens, like digoxigenin or biotin.

Said alkyl, alkenyl and alkynyl groups can be unsubstituted or substituted by one or more groups with the proviso that said groups are chosen from a group consisting of -halogeno, -SH, -S, -(C1-C8)-alkyl, -(C1-C8)-alkoxy, -OH, NR¹⁵R₁₆, -C(=O)R¹⁷, -NH-C(=O)NR¹⁵R¹⁶, -NH-C(=S).

Said alkyl, -alkenyl or alkynyl groups can be either arranged in linear, branched or cyclic form. In case of NR¹⁴R¹⁵, R¹⁴ and R¹⁵ can be chosen in such a way that they form together with the nitrogen atom a 5 or 6 membered ring.

In the scheme below (Scheme 3B), specific embodiments called special head groups, SHG, having N-heterocyclic bases are shown. Said N-heterocyclic bases are particular preferred embodiments of the nucleobase moiety.

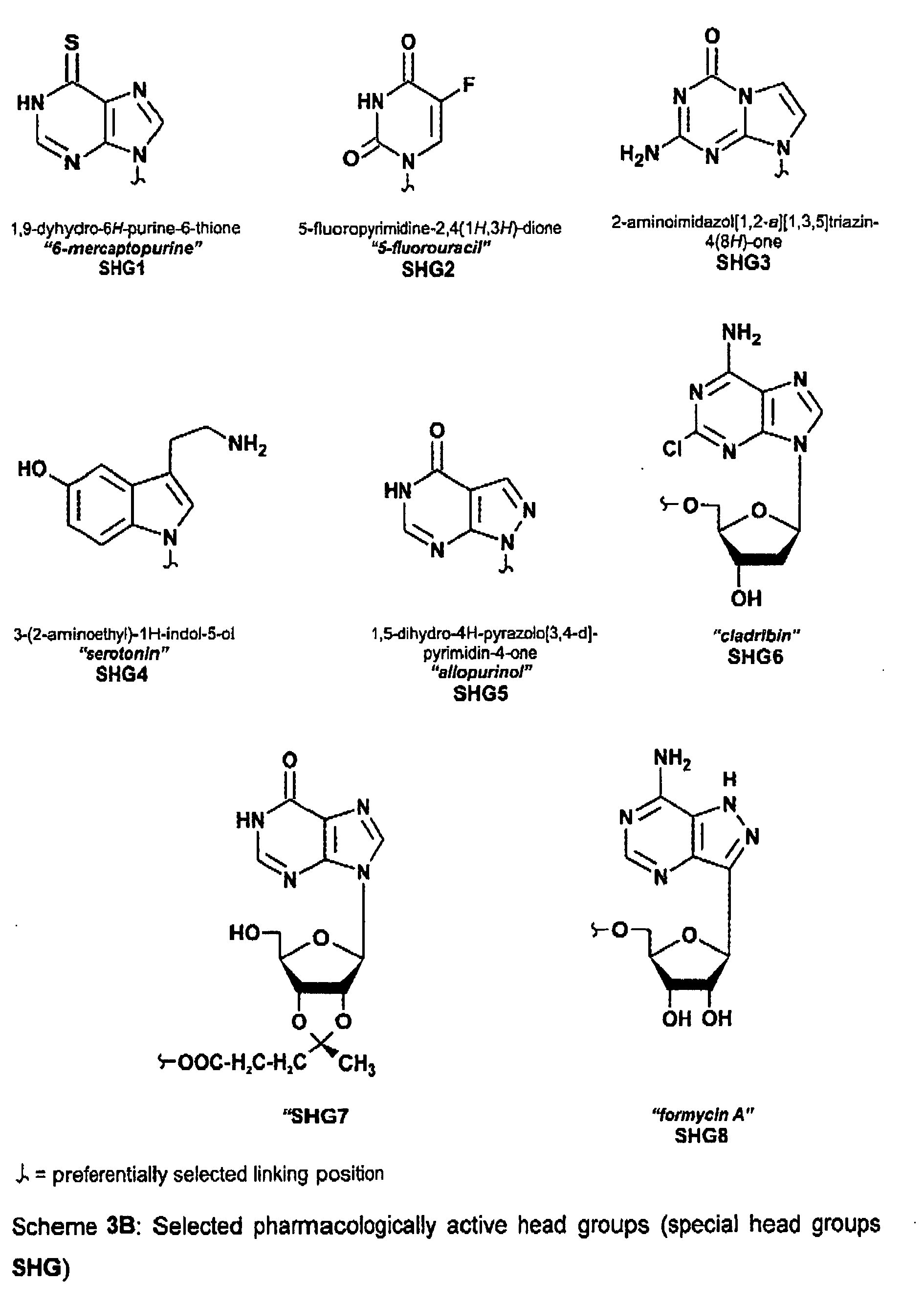

The synthesis of the N-heterocyclic nucleobase moieties shown in the scheme above are well known to the skilled person. Said compounds are also commercially available. For example methods for the synthesis of said compounds are described in R. J. Suhadolnik, Wiley-New York, 1979; F. Seela, H. Rosemeyer, in: "Recent Advances in Nucleosides, Chemistry and Chemotherapy" (Ed.; Chu C. K.), Elsevier 2002.

In the following scheme (Scheme 3C) further N-heterocyclic moieties are shown. These N-heterocyclic moieties represent examples of SHG representing pharmacologically active groups.

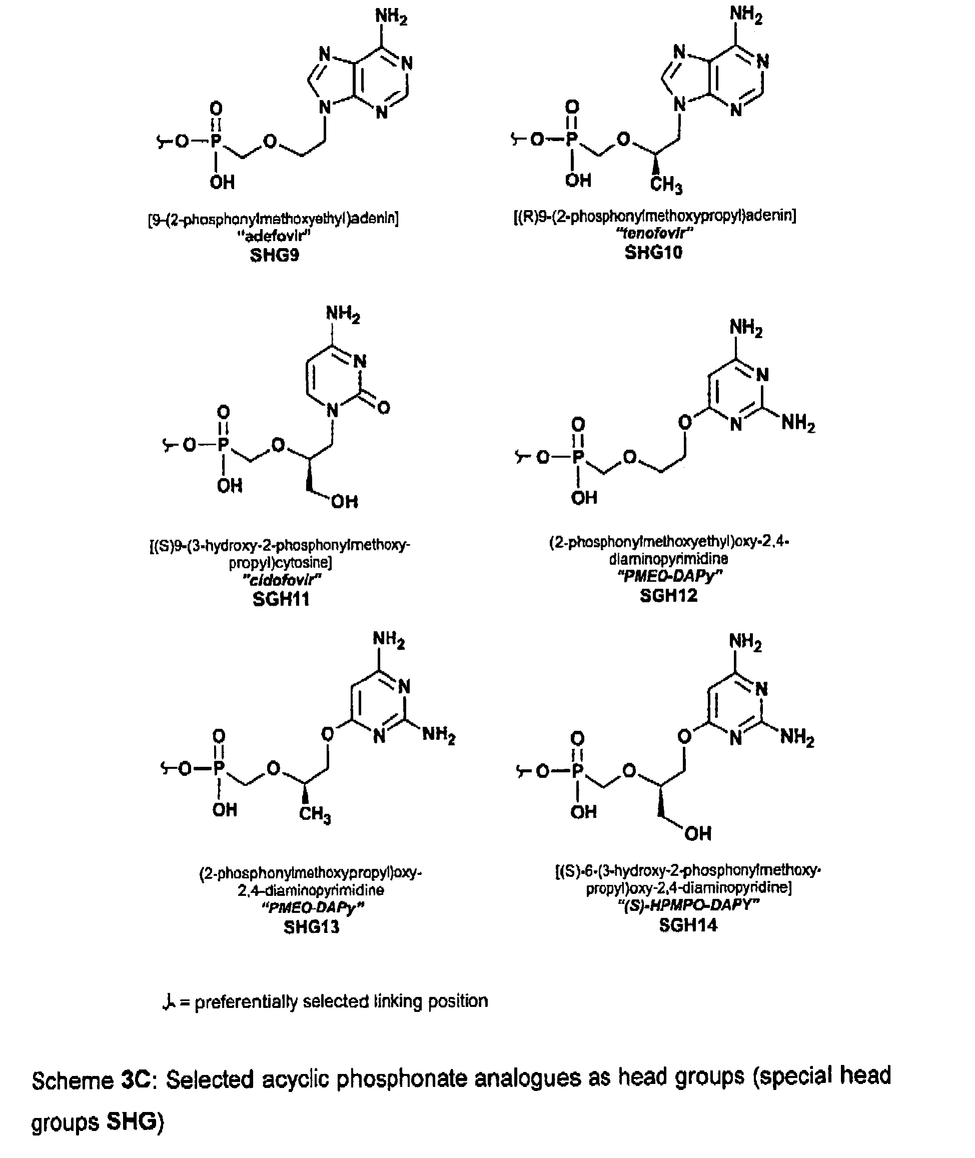

### Spacer moieties

The spacer moieties refer to a part or component of the claimed hybrid compound which connects the base moiety with the redox active moiety. Preferably, the spacer is selected from the group consisting of a linear or branched alkyl-, alkenyl -or alkynyl-group having 1 to 10 C-atoms, an aryl-group or a (CH₂CH₂-O)ₙ-group with n being an integer of 1 to 100. Preferably, the alkyl-, alkenyl- or alkynyl-group has 1 to 6 carbon atoms, more preferably 1 to 4 carbon atoms. The aryl group may be e.g. a benzene group or a phenyl group. The spacer moiety has at least two reactive groups preferably being located at the terminal ends of the spacer moiety. Said terminal reactive groups may be identical or may be different. Preferably, said reactive group is a halogen substituent, like chloride, bromide or iodide. In a another preferred embodiment the spacer moiety is a disubstituted aromatic compound like 1,4-dibromomethylbenzene. The reaction of said spacer moiety containing two terminal reactive groups with the N-heterocyclic base or the special head group containing the N-heterocycle is shown in the scheme below.

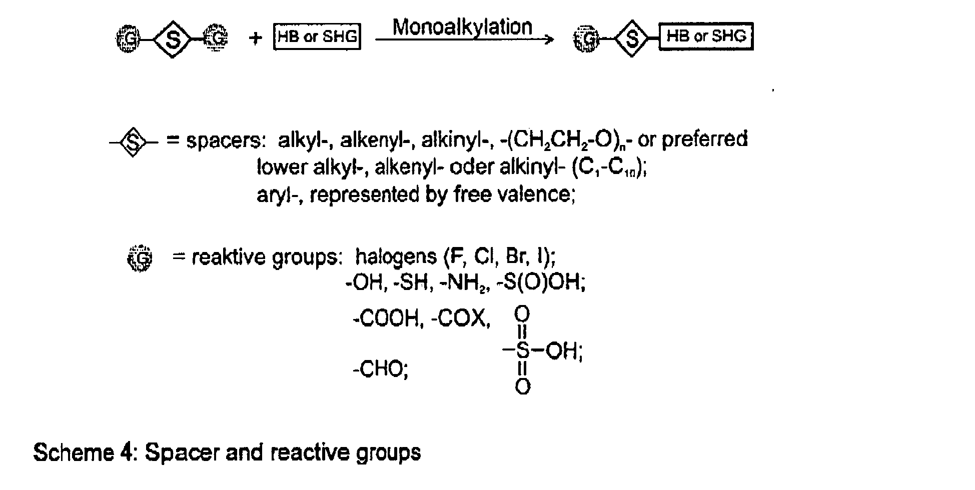

The activated spacer moieties containing two reactive groups as shown above are reacted with the base moiety for monoalkylation, thus, obtaining a reactive intermediate compound, consisting of the base and the spacer molecule having terminal a reactive group. To allow alkylation of the base molecules strong basic conditions are necessary. Normally, the redox active moiety like the viologen moiety, will be reduced or degraded in a highly basic environment. Thus, it is necessary to react said base moiety with the spacer first and subsequently react the intermediate compound composed of the spacer moiety and the base moiety with the redox active moiety.

The spacer moiety allows expanding the conjugated Π-electron system of the moiety having redox activity. In other embodiments the spacer molecules allow decoupling the nucleobase moiety from the Π-electron system of the moiety having redox activity, if desired. For example, in case of using spacer moieties of C1 to C2 alkyl-or alkenyl groups an electronic resonance may be achieved between the redox active moiety and the base moiety(ies). Thus, varying the spacer moiety and/or the nucleobase moiety(ies) allows to control the colour of the electrochromic compound.

Preferably, the spacer is linked with the nucleobase moiety via an N-atom of the nucleobase moiety; for example in case of a purine nucleobases the spacer is linked to the purine nucleobase at position 9 or in case of a pyrimidine nucleobase with the N-atom at position 1. In another embodiment, the spacer moiety is linked to the nucleobase at a C-atom of said nucleobase by C-alkylation. Furthermore, when special head groups are used, linkage between the spacer moiety and the base moiety may also be a covalent linkage via an O or S atom.

### Redox active group

The redox active group may be any redox active group shown in the scheme below. 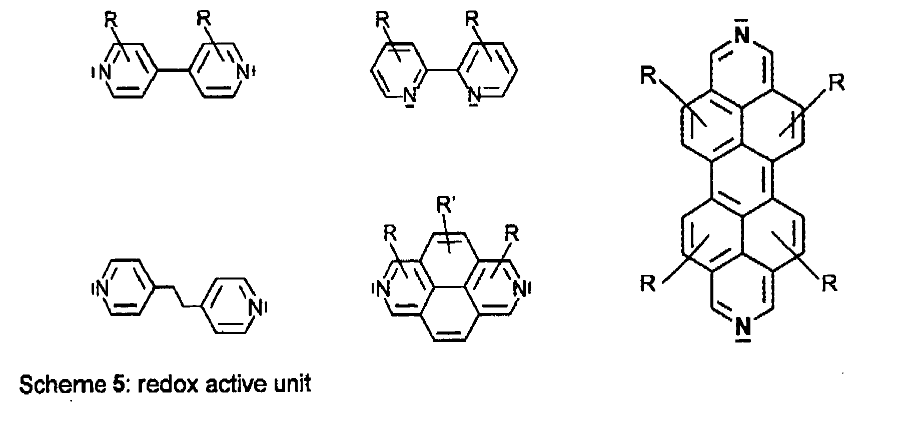 That is, the redox active group is preferably a 4,4'-dipyridine, a 2,2'-dipyridine, a 1,2-di(4-pyridyl)ethane, a 2,7-diazapyrene and a 2,9-diazaperylene or derivatives thereof which may be substituted once or several times. In scheme 5 R may be defined as R1 to R12 in scheme 3a.

In a preferred embodiment the redox active group is 4,4'-bipyridine. Characterization of the 4,4'-bipyridine and 2,7'-diazapyrene moiety as electrochromic compounds can be found for example in: F. Würthner, A. Sauter and J. Schilling, "Synthesis of Diazadibenzoperylenes and Characterization of Their Structural, Optical, Redox, and Coordination Properties", J. Org. Chem., 2002, 67, 3037-3044.

Compounds such as diazapyrene, diazaperylene, bipyridine, 1,2-di(4-pyridyl)ethane may restrict the conjugated Π-electron system to the redox active moiety or may expand said conjugated Π-electron system to the heterocylic nucleobases depending on the spacer moieties and the substitutents used. The extension of the conjugated Π-electron system influences the adsorption spectrum (colour) of the compounds as well as of the redox potential of the compounds. For example, while dialkyl substituted bipyridinium salts or benzyl substituted bipyridinium salts are blue, diaryl substituted bipyridinium salts are green.

The strategy for the synthesis of the new hybrid compounds according to the present invention may be different - depending on the later application of said hybrid compounds.

In the field of electrochromism the coupling of said compounds onto the surface of a support material in a cascade fashion is desired. For this purpose the hybrid units may be synthesized in advance and may subsequently be coupled with additional core units and, said units may the be coupled to nucleophilic or electrophilic anchor groups allowing later linkage to mesoporous support materials. Said support materials may be, for example, In₂O₃/SnO₂ = ITO; F/SnO₂=FTO; Sb₂O₃/SnO₂=ATO; TiO₂, Au, mesoporous silicates M41 S, like MCM41 or MCM48, zeolites etc.

In another embodiment, the synthesis results in the preparation of dendrimers. When synthesizing dendrimers, functional molecules of the cascade type are build, wherein the redox active moiety and the redox active periphery are sterically and/or electronically isolated from each other. Depending on the structural build-up, the dendrimers may be divided into two groups: i) the base as a functional unit is located in the core of the dendrimer and ii) the base is located at the periphery or located throughout the dendritic scaffold.

For example, said hybrid molecules may be linked to a solid layer like a semiconductor nanocrystalline TiO₂ in monomolecular layers via alkylphosphonate group as coupling group. The TiO₂ nanocrystals are sintered on an indium oxide layer. These layers result in the production of transparent electrodes which can change their colour fast and efficiently upon changing the charge of said electrode.

Further, hybrid compounds having additionally a functional group, may be synthesized; said hybrid compounds may be used for stabilizing complex nucleic acid structures, e.g. G-tetrades.

Furthermore, the hybrid units may be used for the preparation of marker molecules for detection of nucleic acids.

The compound according to the present invention may be composed of at least two nucleobase moieties which may be identical or different from each other. In case of having at least two nucleobase moieties said hybrid compounds may be present as symmetrical hybrid compounds or asymmetrical hybrid compounds. In case of symmetrical hybrid compounds the nucleobase moieties are identical or may be different whereas in case of asymmetrical hybrid compounds, a unidirectional substitution occurs where a nucleobase is present on one side of the redox active moiety and a second group, e.g. an anchor group like methoxysilanyl, ethoxysilanyl, epoxysilanyl, phosphonate, salicylat, benzoate, benzoyl residues or alkyl-, benzyl-, or aryl residues may be present on the other side. According to the present invention there are provided compounds of the general structure i) or ii) as shown in the scheme below (Scheme 6).

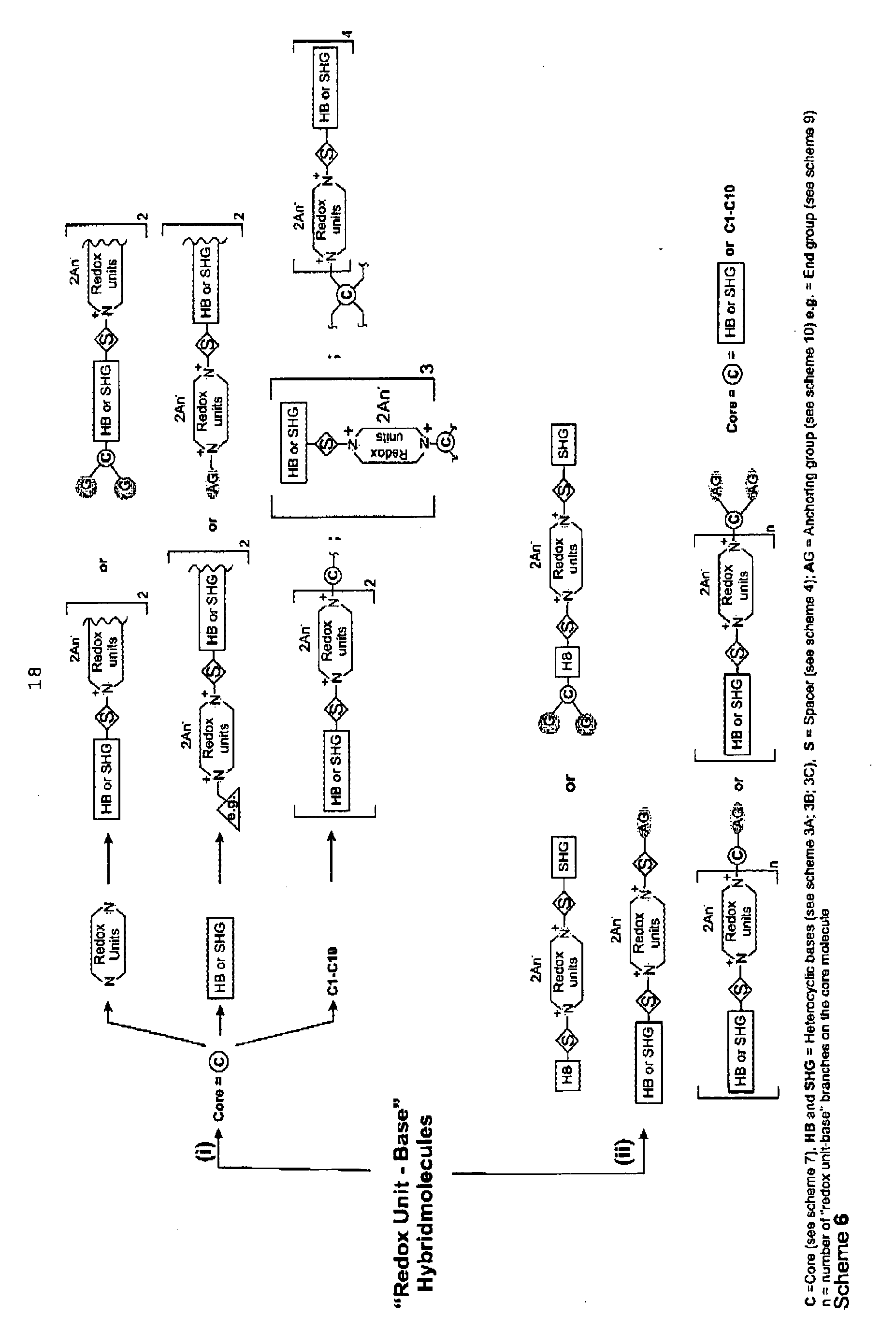

In route i) of Scheme 6 examples for symmetrical compounds are shown. That is, two of the hybrid units are covalently linked to a core moiety as will be discussed below in more detail. In route ii) of Scheme 6 examples for asymmetrical compounds are shown.

In case of asymmetrical hybrid compounds having a nucleobase, a spacer, and a redox active moiety, said hybrid units are not coupled to a common core moiety, but contain at the redox active moiety a group which allows the coupling with other materials for example with support materials. Groups for coupling of the hybrid unit are e.g. alkylphosphonic acid, benzoic acid, salicylic acid or silane derivative allowing chemical linkage to the material. Derivatization of a surface is performed layer by layer using alternately electrophilic and nucleophilic functional units. This allows to build-up 2D or 3D polymeric films on the surface.

### Core molecules/core moieties

The hybrid compounds or hybrid units described above may be covalently linked to core moieties, thus, forming asymmetrical unidirectional molecules or symmetrical bidirectional or multidirectional dendrimers of 0^{th} order. Embodiments of said core moieties are shown in the following scheme (scheme 7).

The core moiety is preferably connected with at least one or more connector moieties preferably having terminally electrophilic or nucleophilic groups, e.g. a halogen atom or an amino group. These reactive connector moieties allow later the linkage to the redox active moiety of the hybrid unit. In the scheme below, the connector moieties are illustrated as alkyl chains. However, they are not restricted thereto. The core moieties have preferably one, two, three or four connector moieties which, in case of aromatic core moieties, represent symmetrical groups of the type; C1, C3, D2H, D3H and, in case of aliphatic cores, symmetrical groups S4.

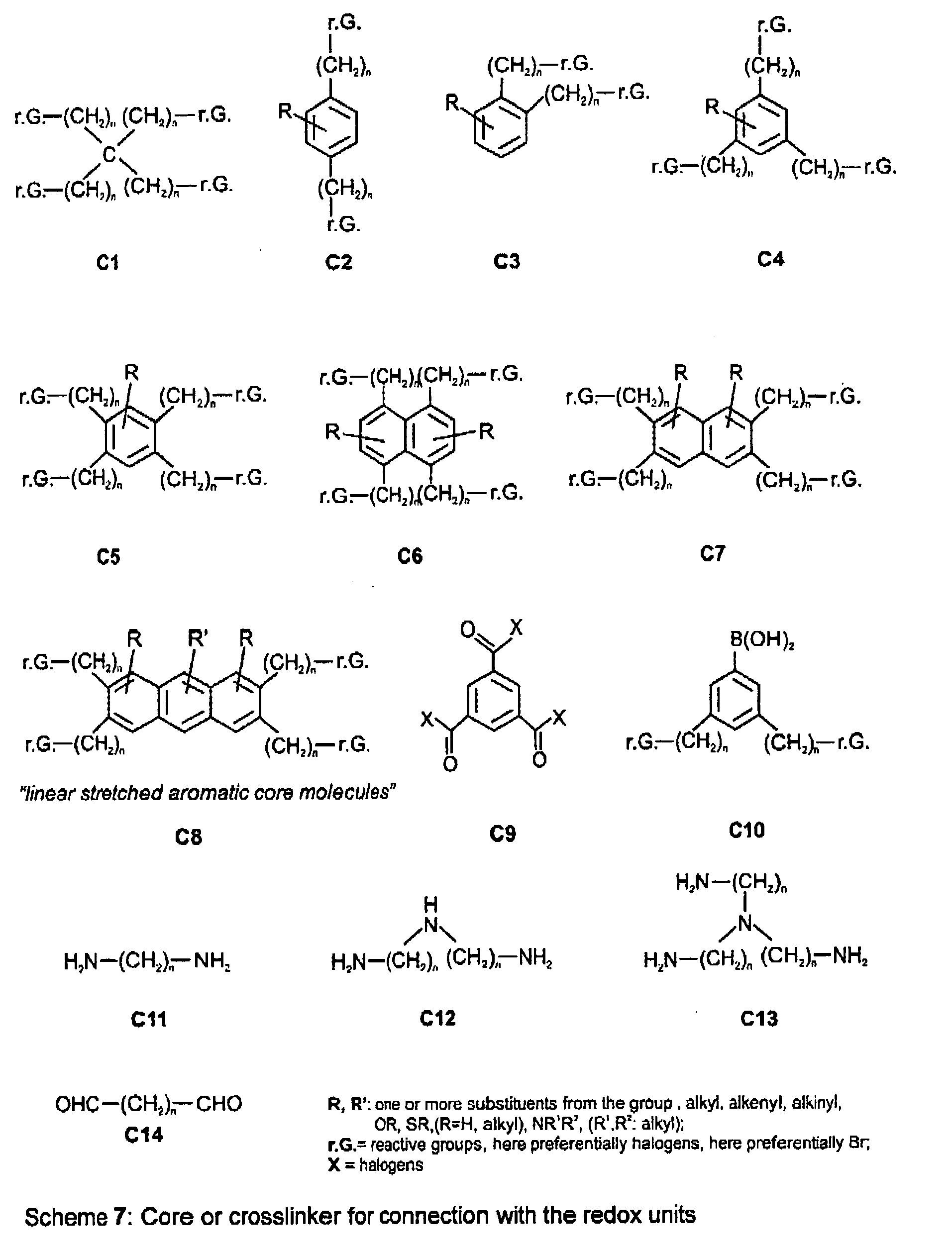

When using core moieties having initiator activity bi- or multidirectional dendritic hybrid molecules may be build-up (0^{th} order), analogous compounds may be obtained using linear core moieties of the aromatic type as shown in the scheme below (Scheme 8).

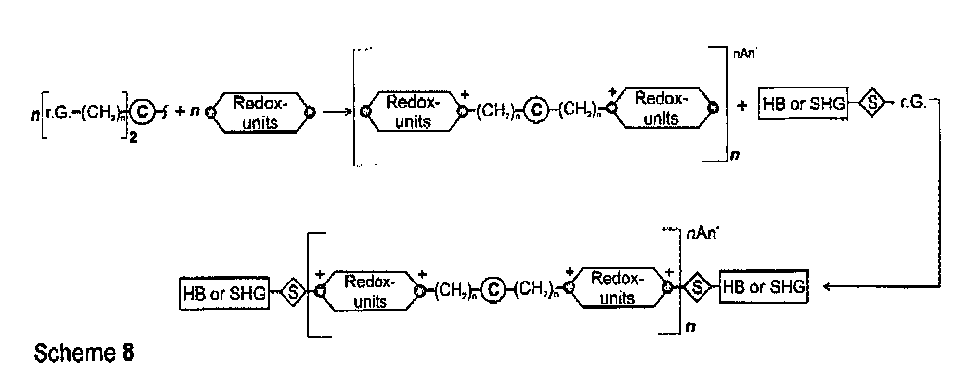

The cascade-like molecules may be synthesized either by starting from the core, the initiator-core to the periphery (divergent synthesis) or starting from the dendritic branches to the core (convergent synthesis). By repeating the synthesis steps, a multiplication of the number of branches and of the number of terminal groups may be achieved. Thus, the next generation of the dendrimers may be obtained. The exponential growth may be terminated using molecules having a single reactive function only, a so-called termination group or end group, see scheme 9.

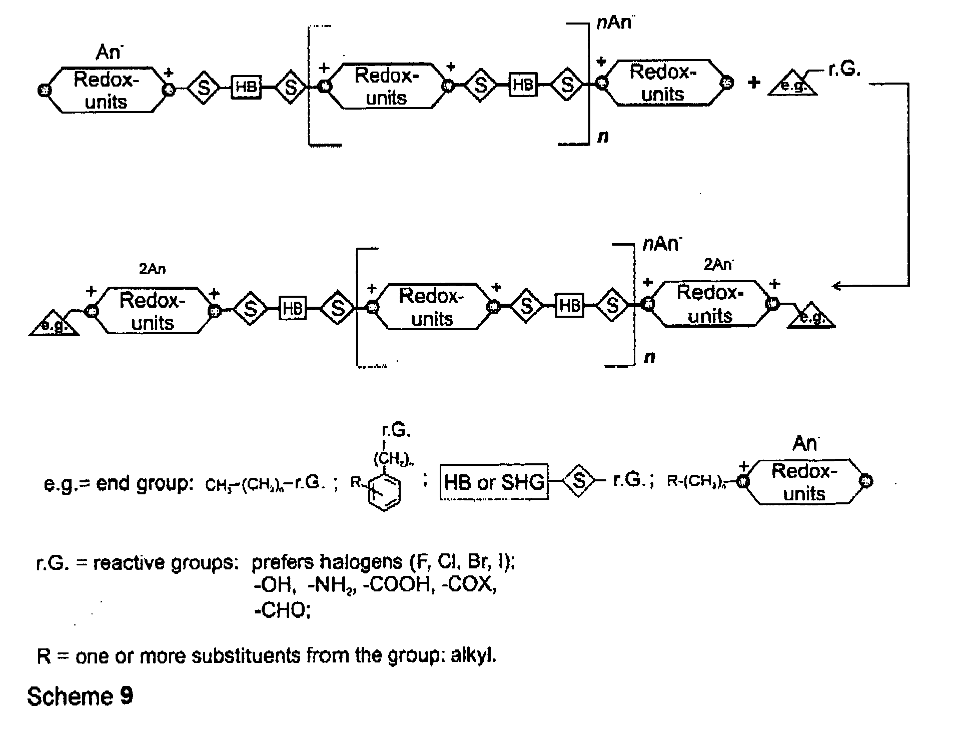

### Hybrid compounds for the covalent linkage to mesoporous support material

In the scheme below (Scheme 10) an example for the synthesis of asymmetrical, unidirectional hybrid compounds is shown which may be coupled via an anchor group to solid support materials in monomolecular layers. The molecules must contain at least one anchor group which may adsorb to a mesoporous support material, like ITO, FTO, ATO, TiO₂, AU, MCM41, zeolites, etc.

Particularly preferred anchor groups are benzoate, salicylate, silane derivatives such as 4-[2-[trichlorsilyl)ethyl]-pyridin, [(chloromethyl)phenyl-ethyl]-trimethoxysilan, (γ-aminopropyl)trimethoxysilan, (γ-aminopropyl)triethoxysilan, [(γ-aminopropyl)-dimethyl)]-ethoxysilan, (δ-aminobutyl)-triethoxysilan, [(γ-aminopropyl)diisopropyl]ethoxysilan, vinyltrimethoxysilan, γ-glycidoxypropyltrimethoxysilan, thiol derivatives. Further preferred is a phosphonate group as anchor group, in particular for TiO₂-material.

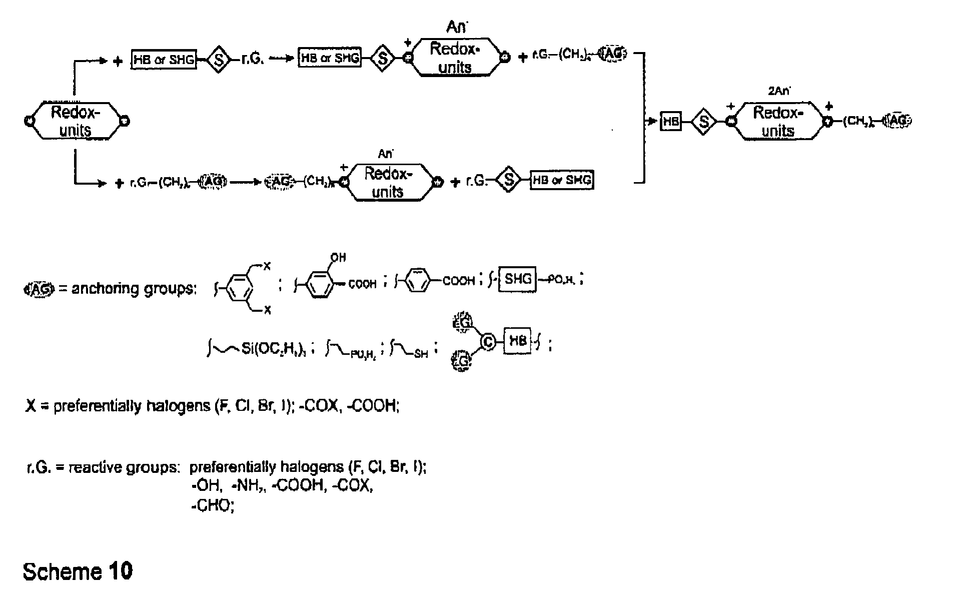

By using these hybrid units, various types of structures may be formed. That is, it is possible to have variations in the number of hybrid units as well as in the number of anchor groups. Furthermore, it is possible to obtain compounds having unidirectionally one or two anchor groups or which have a dendritic structure containing multiple anchor groups.

Compounds containing a bipyridine moiety with a phosphonate residue are described generally e.g. in WO2004/067673. However, the specific compounds according to the present invention having a phosphonate anchor group are not exemplied therein.

As support materials, materials such as TiO₂ or ATO are suitable since these materials have surface areas being up to 100-fold larger than the surface area of ITO or FTO glass. For example, these substrates or support materials for electrodes are sintered TiO₂ anatase or fluorine tin oxide glass (FTO) (150 °C). These types of substrates for electrodes have mesopores with a diameter of 10 to 30 nm. Hybrid molecules according to the present invention containing anchor groups are linked with the surface of said materials, and, if desired, are cross-linked. Thus, transparent electrodes which allow an efficient and fast colour change due to a change of its charges may be obtained. In case of using spacer moieties with C1 and C2 units, electronic coupling between the redox active moiety and the base moiety is achieved. Thus, a variation the colour of the electrochromic unit by varying the nucleobase may occur.

To allow layer-by-layer covalent fixation of an alternate redox active moiety and a nucleobase moiety on the surface, compounds as shown in the scheme below (Scheme 11) may be used, the synthesis of said compounds is shown in scheme 10. The general synthesis of said compounds is shown in scheme 12, scheme 13 represents an example of the generalized scheme.

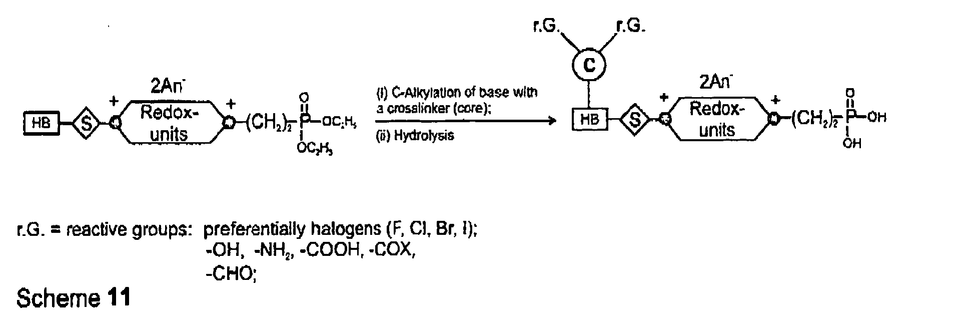

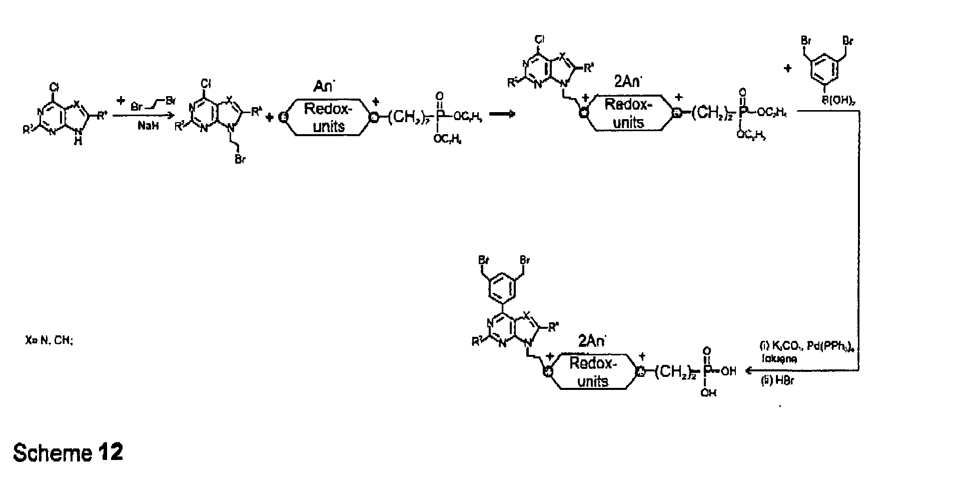

For the preparation of hybrid molecules comprising the base moiety, the redox active moiety and the anchor group, e.g. an alkyl phosphonic acid diester as shown in scheme 10 or a Suzuki-Miyaura reaction (Scheme 12) may be performed linking a 1,3-di(bromomethyl)phenyl branching molecule containing two reactive groups to the heterocyclic base moiety. Compounds are obtained after performing acid hydrolysis of the phoshonic ester group which may be fixed to a mesoporous support material, e.g. a TiO₂ surface, thus, allowing the immobilization of electroactive compounds on mesoporous electrodes.

This is achieved by forming a self-organizing monolayer on the inner surface of a mesoporous support material. This layer may be cross-linked and may be build-up in direction to the centre of said cores by substitution, condensation or electropolymerisation reactions (see for example Ph. D. thesis, Asaftei C.S., Universität Osnabrück, July 2005). This cascade-like reactions allows the possibility of forming redox active films with a high memory capacity on the electrodes, thus, allowing to use the same in the field of biosensors.

A further preferred embodiment of the compounds according to the present invention is shown in schemes 13 and 14 below, wherein a trimethoxysilyl alkyl group is used as anchor group instead of an alkyl phosphonate. The nucleobase may have a crosslinker C, as shown in schemes 13 and 14. The synthesis of the spacer and the 2,7-diazapyrene having terminal reactive groups is described e.g. in Deventers N. et al., Chem. Mat. 1999, 11, 2837.

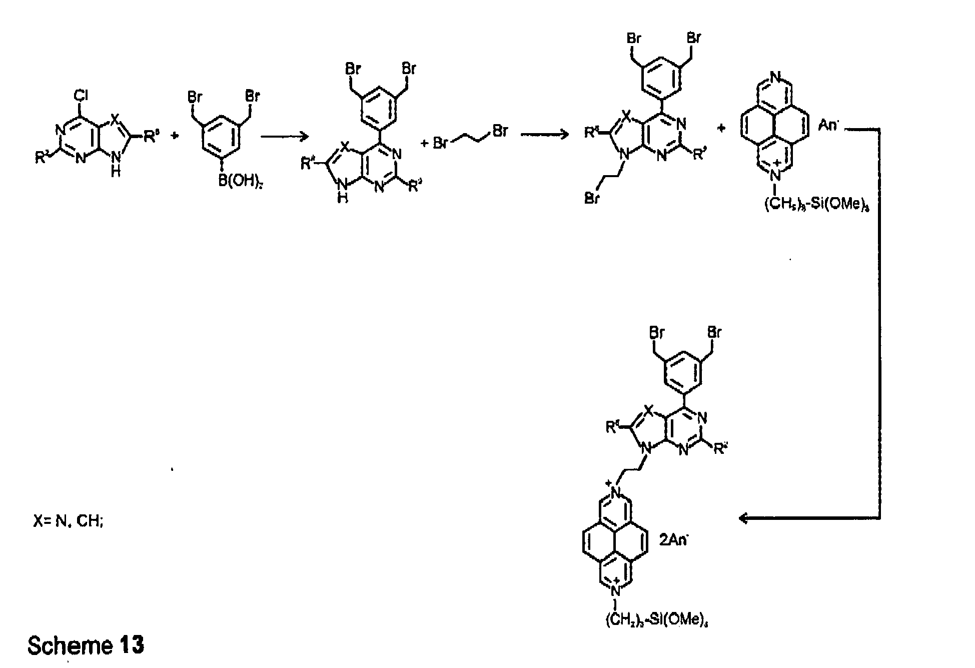

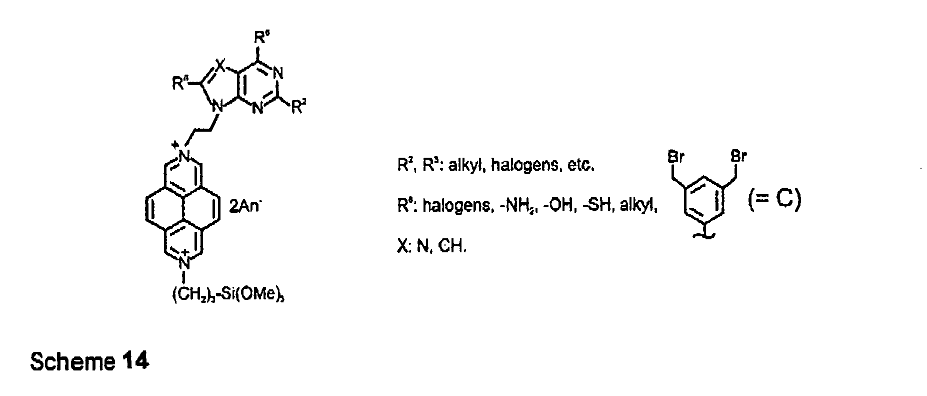

### Hybrid compounds obtained by C-alkylation of the nucleobase moiety.

Schemes 15A and 15B shows the synthesis of hybrid molecules according to the present invention, wherein the covalent linkage between the heterocyclic base moiety and the redox active group via a spacer moiety is achieved by C-alkylation of the base. In this case, the first step of synthesis is a carbon-carbon linkage between a suitable nucleobase moiety derivative, e.g. a derivate having a iodide group and an alkyl-, alken-1-yl- or alkin-1-yl- compound having a terminal reactive group, preferably, a halogen substituent (see schemes 15A, 15B). The methods used for the synthesis are known to the skilled person and are described e.g. in F. Seela, N. Ramzaeva, H. Rosemeyer, Science of Synthesis, Product Class 17: Purines, Vol. 16, pp. Derivates according to the present invention may also be obtained from the nucleobases shown in scheme 3A above.

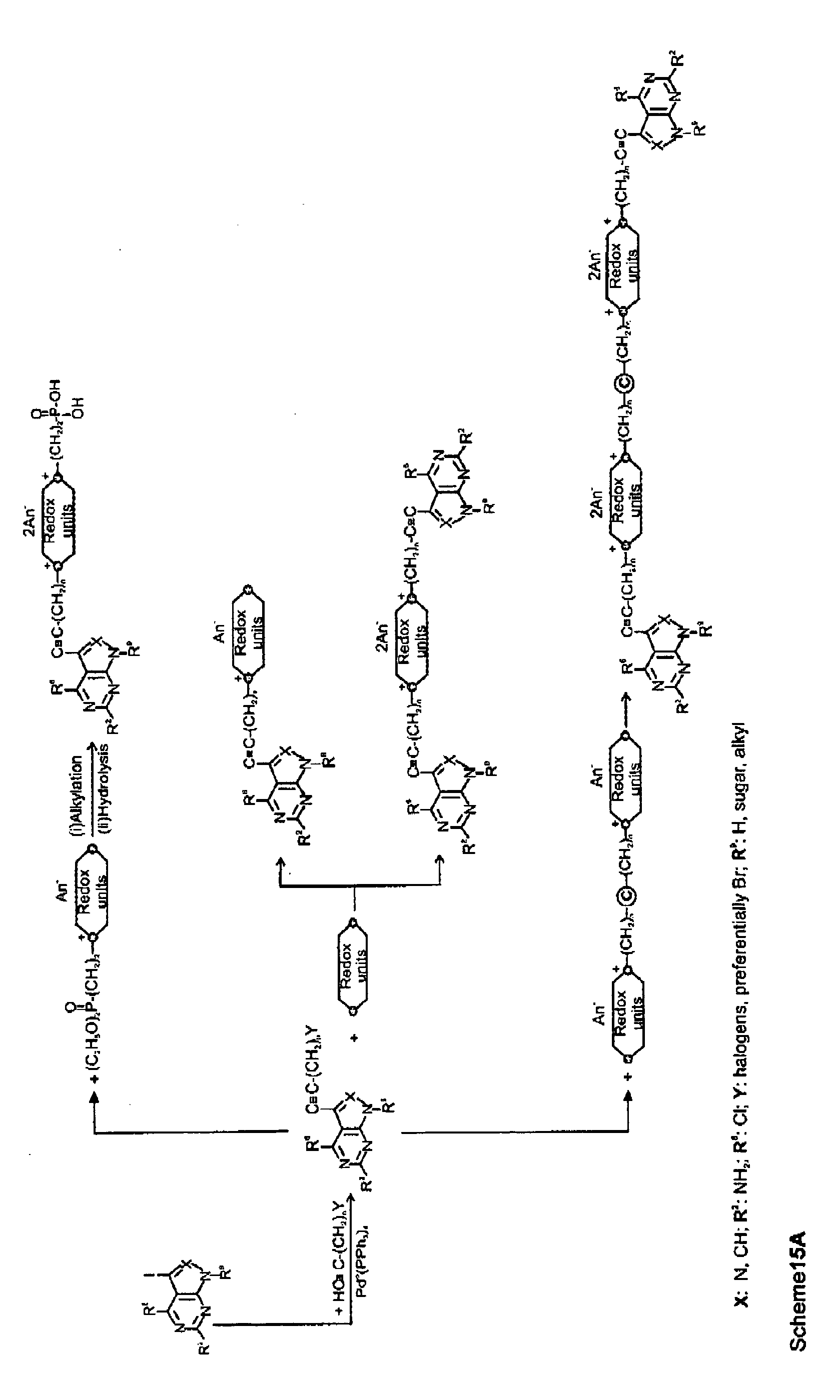

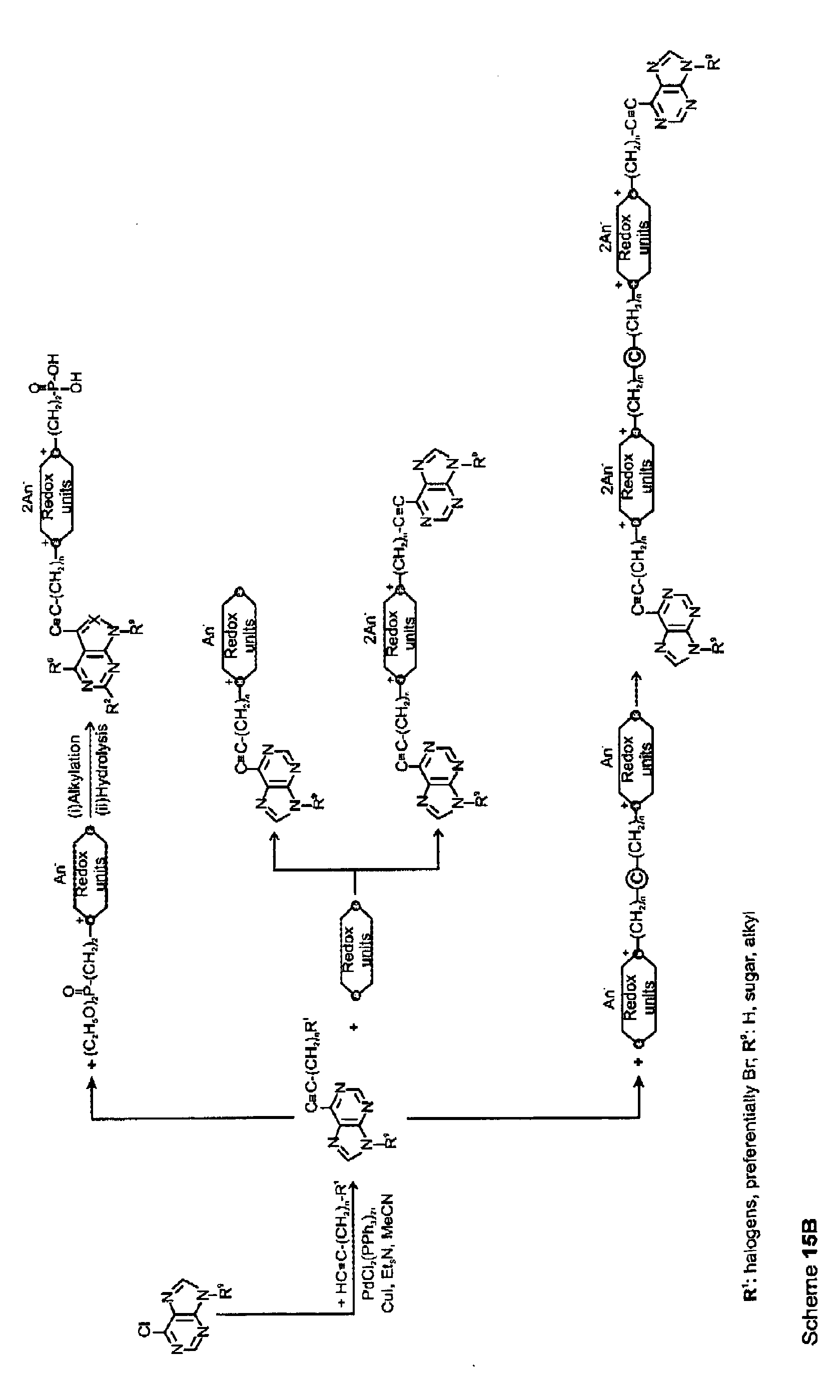

### Field of the application

The hybrid compounds according to the present invention may be used in various fields, for example as electrochromic compounds. Moreover, the compounds according to the present invention may be used as nucleosides as well as compounds having antibacterial, antiviral and antifungal activity. That is, the compounds of the present invention may be the active principle of pharmaceutical compositions, in particular of pharmaceutical compositions for treating infectious diseases, e.g. viral disease, such as HIV-1 or HIV-2 infection, HSV-infection, SIV or FIV-infection, or infection with the coxsackie virus.

Furthermore, C-alkylated nucleobases allow using said nucleobases as a part of nucleosides and nucleotides. Said nucleosides and nucleotides may be used in nucleic acid synthesis methods known in the art. For example, said nucleotides may be incorporated into oligonucleotides and said oligonucleotides may be used as probes in nucleic acid- based detection methods, such as diagnostic methods, etc.

The hybrid compounds according to the present invention will be described by way of examples in the following without limiting the present invention thereto. The examples are provided for illustrated purposes only and the skilled person is well aware of any modification changes etc. without departing from the scope of the claimed subject matter.

### Examples

### Synthesis of compounds according to the present invention

### Example 1

### Synthesis of 7-(2-bromoethyl)-4-chloro-7H-pyrrolo[2,3-d]pyrimidine

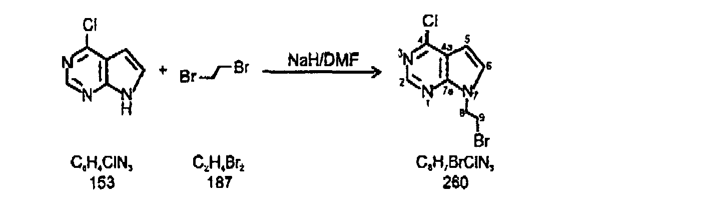

4-Chloro-*7H*-pyrrolo [2,3-*d*] pyrimidine (2g, 13 mmol) and 4 g of NaH were suspended in 20 ml of freshly distilled DMF and 1,2-dibromoethane (242 g, 1,3 mol) was added in 10 ml DMF. The suspension was stirred at 70°C for 3 days under reflux. The reaction mixture was filtered, and the mother liquor was evaporated. The crude product was dissolved in chloroform and purified by column chromatography (silica gel column, 35 x 3.5 cm, petrolether/ethyl acetate (4:1, v/v) as eluent). The first fraction contained 4-chloro-7-vinyl-7*H*-pyrrolo[2,3-*d*]pyrimidine (R_{f} = 0.67) and the second fraction the title compound 7-(2-bromoethyl)-4-chloro-7*H*-pyrrolo[2,3-*d*]pyrimidine (R_{f} = 0.42). The product-containing fractions were pooled, and the solvent was evaporated. After drying in vacuo (r.t., 24 h) 2.15 g (8.2 mmol, 63 %) of *7-(2-bromoethyl)-4-chloro-7H-pyrrolo[2,3-d]pyrimidine* was obtained as a light yellow powder.
- **¹H-NMR** (500 MHz, CDCl₃):: δ(ppm) = 8.58 (s, 1H, H-C(6)), 7.34 (t, ³J [H,H] = 5.0 Hz, 1 H, H-C(2)), 6.55 (d, ³J[H,H] = 2.5 Hz, 1H, H-C(5)), 4.67 (t, ³J[N,H]=5.0 Hz, 2H, H-C(8)), 3.76 (t, ³J[H,H] = 5.0 Hz, 2H, H-C(9)).
- **¹³C-NMR** (500 MHz, CDCl₃):: δ(ppm) = 150.09 (C2), 149.68 (C7a), 144.16 (C4), 129.74(C6), 121.11(C4a). 100.82 (C5), 46.97 (C8), 30.00(C9).

### Example 2

### Synthesis of 2-amino-7-[bromomethyl]-4-chloro-7H-pyrrolo [2,3-d]pyrimidine.

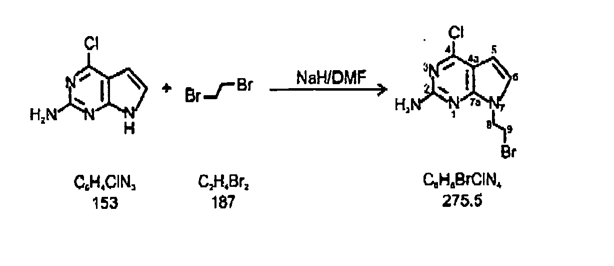

2-Amino-4-chloro-*7H*-pyrroo[2,3-*d*] pyrimidine (2,2g, 13 mmol) and 4 g of NaH were suspended in 20 ml of freshly distilled DMF and 1,2-dibromethane (242 g, 1.3 mol) was added in 10 ml of DMF. The suspension was stirred at 70°C for 3 days under reflux. The reaction mixture was filtered, and the mother liquor was evaporated. The crude product was dissolved in chloroform and purified by column chromatography (silica gel column, 35 x 3,5 cm, petrolether/ethyl acetate (4:1, v/v) as eluent). The first fraction was discarded, and second fraction containing the title compound 2-amino-7-(bromoethyl)-4-chloro-7*H*-pyrrolo[2,3-*d*]pyrimidine (R_{f} = 0.42) was collected, and the solvent was evaporated. After drying in vacuo (r.t., 24 h) 1.84 g (6.6 mmol, 51.4 %) of the title compound was obtained as a yellowish powder.
- **¹H-NMR** (250 MHz, d₆-DMSO):: δ(ppm) = 7.22 (d, ,³J [H,H] = 5.0 Hz, 1H, H-C(6)), 6.71 (s, 2H, H₂N), 6.30 (d, ³J[H,H] = 5.0 Hz, 1H, H-C(5)), 4.41 (t, ³J[N,H] = 12.5 Hz, 2H, H-C(8), 3.83 (t, ³J[H,H] = 12.5 Hz, 2H, H-C(9)).
- **¹³C-NMR** (250 MHz, d₆-DMSO):: δ(ppm) = 159.33 (C2), 153.54 (C7a), 151.21 (C4), 126.35 (C6), 108.63 (C4a), 98.55 (C5), 45.35 (C8), 31.30 (C9).

### Example 3

### Synthesis of 1-{2-[4-chloro-7H-pyrolo[2,3-d]pyrimidine-7-yl]ethyl]}-1'-[(2)diethoxy-phosphoryl)ethyl]-4,4-bipyridinium dibromide.

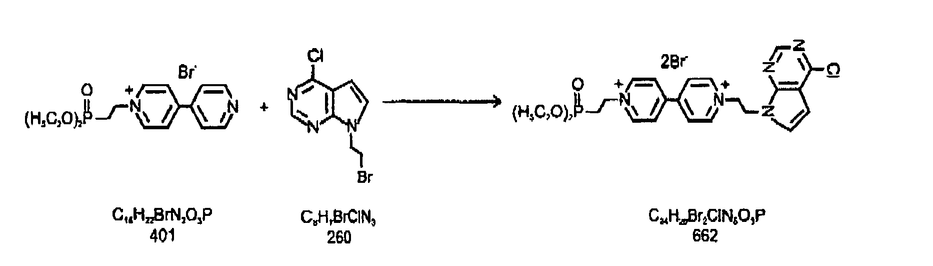

1-[2-(Diethoxyphosphoryl)ethyl]-4-pyridine-4-pyridinium bromide (87 mg, 0.21 mmol) was dissolved in 10 ml of MeCN and 89.5 mg (0.34 mmol) of 7-(2-bromoethyl)-4-chloro-*7H*-pyrolo[2,3-d]pyrimidine were added. The mixture was refluxed for 14 days. After three days a first crop of crystals was formed. After cooling the reaction mixture a brown solid was filtered off, washed with ether and evaporated to dryness. The mother liquor was evaporated to dryness, and the residue was dried followed by resolving in MeOH and recrystallisation with ether overnight at 4 °C. After evaporation to dryness for 24 hours, 89.4 mg (0.13 mmol, 64 %) of a yellowish powder was obtained.
- **¹H-NMR** (250 MHz, D₂O):: δ(ppm) = 9.06 (t, ³J [H,H]= 15.1 Hz, H2), 8.68 (t, ³J [NH]= 12.0 Hz, 1H), 8.33(dd ,³J [N,H]= 5.0 Hz, ³J [H,H] = 7.5 Hz, 2H), 8.22(d, ³J [H,H]= 5.0 Hz, 2H), 8.13(q, ³J [H,H]= 6.7 Hz, 2H), 7.47 (q, ³J [H,H]= 10.0 Hz, 1H), 6.72 (s, 1H), 5.09 (s, 2H), 4.89 (s, 2H), 4.78 (t, ³J [H,H] = 10.0 Hz, 2H), 4.01 (q, ³J [H,H] = 5.0 Hz, 4H), 2.29 (t, ³J[NH]=7.5 Hz, 2H), 1.11 (t, ³J[H,H] = 7.5 Hz, 6H).
- **¹³C-NMR** (250 MHz, d₆-DMSO):: δ(ppm) = 154.5 (C7a-B), 152.4 (C4-B), 151.1 (C4'-Bipy), 150.5 (C2-B), 149.7 (C4-Bipy), 146.2 (CH-2',3'-Bipy), 145.8 (CH-4',5' Bipy), 130.8 (C6-B), 127.3 (CH-2,3-Bipy), 127.0 (CH-4,5-Bipy), 117.7 (C4a-B), 101.6 (C5-B), 64.2 (C1-Ethyl-Phosph-C-N, ²J = [C,P] = 6.5), 61.5 (C1-Ethyl-B), 58.2 (C1-Ethyl-Phosph-O-C), 45.5 (C2-Ethyl-Base), 29.3 (C2-Ethyl-Posphonat-P-C), 26.1 (C1-Ethyl-Phosph-P-C, ¹J[C,P] = 133,7 Hz), 16.1 (C2-Ethyl-Phosphonat-O-C, ³J[C,P] = 5,6 Hz).

### Example 4

### Synthesis of 7-(2-chloroethyl)-2-methylthio-7H-pyrrolo[2,3-d]pyrimidin-4-one

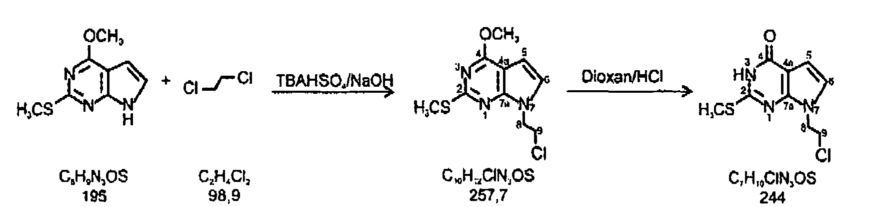

1. Step: 4-Methoxy-2-methylthio-7*H*-pyrrolo[2,3-*d*]pyrimidine (500 mg, 2.56 mmol), suspended in dichloroethane (20 ml), was added to a solution of tetrabutylammonium hydrogenesulfate (0.7 g, 2.5 mmol) in 50% aq. sodium hydroxide and agitated with a vibromixer for 3h at room temperature. After separation of the layers, the aqueous phase is extracted twice with dichloromethane. The combined organic layers were evaporated to dryness, dissolved in ethanol, decolorized with charcoal and filtered. Addition of water yields colorless needles (175 mg) with a m.p. of 86-87 °C. Column chromatography of the solid (silica gel, CH₂Cl₂) gave two zone, the first of which contained a bis-ethylene by-product. From the slower migrating zone 7-(2-chloroethyl)-4-methoxy-2-methylthio-7*H*-pyrrolo[2,3-*d*]pyrimidine title compound (155 mg, 24 %) was isolated and crystallized from EtOH/H₂O.
   m.p. 90°C.
   TLC (silica gel, CH₂Cl₂): R_{f} 0.76.
   UV (MeOH): λₘₐₓ 282, 238 nm (ε, 13.88, 19.300).
   Anal. Calcd. for C₁₀H₁₂N₃OSCl (257.747): C, 46.60%; H, 4.69; N, 16.30. Found: C, 46.77; H, 4.81; N, 16.35.
   - **¹H-NMR** (250 MHz, CDCl₃):: δ(ppm) = 2.61 (3H, s, SCH₃); 3.87 (2H, t, CH₂N, J = 6.0 Hz); 4.10 (3H, s, OCH₃); 4.50 (2H, t, CH₂Cl, J = 6.0 Hz); 6.43 (1 H, d, H-C(5), J = 4.0 Hz); 6.94 (1H, d, H-C(6), J = 4.0 Hz).
   - **¹³C-NMR** (250 MHz, CDCl₃):: δ(ppm) = 14.3 (SCH₃); 42.7 (CH₂Cl); 47.0 (CH₂N); 53.72 (OCH₃); 99.1 (C-5); 102.9 (C-4a); 125.2 (C-6); 153.6 (C-7a); 163.2 (C-2); 164.6 (C-4).
2. Step: 7-(2-Chloroethyl)-4-methoxy-2-methylthio-7*H*-pyrrolo[2,3-d)pyrimidine (150 mg, 0.58 mmol) was dissolved in a mixture of 50 ml of dioxane and 15 ml of 0.5 N HCl and refluxed for 6 h. After neutralization by adding solid NH₄HCO₃, the solution was evaporated until crystallization started. The precipitate was filtered off and applied to a silica gel column (3.5 x 40 cm). Elution with CH₂Cl₂/MeOH (9:1, v/v) afforded one main zone from which pure 7-(2-chloroethyl)-2-methylthio-7*H-*pyrrolo[2,3-*d*]pyrimidin-4-one was obtained as coloriess crystals (120 mg, 85%);
   m.p. 196°C.
   TLC (silica gel, CHCl₃-MeOH, 9:1, v/v): R_{f} 0.7.
   UV (MeOH): λₘₐₓ 271, 286 nm (ε 10.500, 10.650).
   Anal. Calcd. for C₉H₁₀N₃OSCl (243.72): C, 44.35; H, 4.14; N, 17.24. Found: C, 44.76; H, 4.26; N, 17.64.
   - **¹H-NMR** (250 MHz, CDCl₃):: δ(ppm) = 2.54 (3H, s, SCH₃); 4.01 (2H, t, CH₂N, J = 6.5 Hz); 4.43 (2H, t, CH₂Cl, J = 6.0 Hz); 6.39 (1H, d, H-C(5), J = 4.0 Hz); 7.07 (1H, d, H-C(6), J = 3.5 Hz); 12.16 (1 H, s, NH).
   - **¹³C-NMR** (250 MHz, CDCl₃):: δ(ppm) = 12.8 (SCH₃); 43.3 (CH₂Cl); 46.0 (CH₂N); 101.5 (C-5); 104.4 (C-4a); 122.8 (C-6); 147.5 (C-7a); 154.8 (C-5); 158.6 (C-2).

### Example 5

### Synthesis of 7-(2-Carboxyethyl)-4-chloro-2-methoxy-7H-pyrrolo[2,3-d]pyrimidine

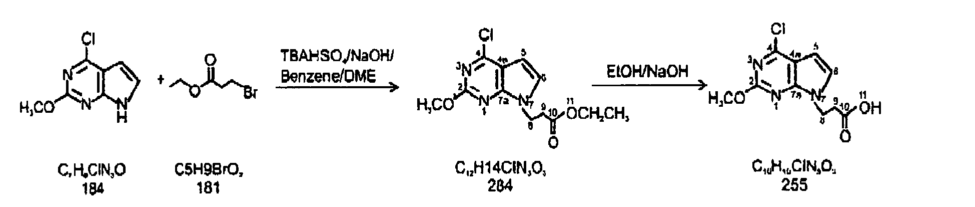

1. Step: 4-Chloro-2-methoxy-7*H*-pyrrolo[2,3-*d*]pyrimidine (500 mg, 2.7 mmol) and tetrabutylammonium hydrogensulfate (92 mg, 0.27 mmol) were suspended in a mixture of 20 ml of 50% aq. sodium hydroxide/10 ml benzene/10 ml dimethoxyethane and agitated with a vibromixer for 5 min at room temperature. Thereupon, ethyl 3-brompropionate (3.35 ml, 27 mmol) was added, and the mixing was continued for 1 h. After separation of the layers the aqueous phase was three times extracted with benzene. The combined organic layers were washed with water, evaporated to dryness, dissolved in MeOH and decolorized with charcoal. Upon addition of a few drops of water the ester 4-chloro-7-(2-ethoxycarbonylethyl)-2-methoxy-7H-pyrrolo[2,3-d]pyrimidine crystallized (635 mg, 83%) as colorless needles.
   M.p. 71-72°C.
   TLC (silica gel, CHCl₃): R_{f} 0.4.
   UV (MeOH): λₘₐₓ 278, 295 nm (ε, 4.300, 5.200).
   Anal. Calcd. for C₁₂H₁₄N₃O₃Cl (283.721): C, 50.80; H, 4.97; N, 14.81. Found: C, 50.94; H, 4.92; N, 14.92.
   - **¹H-NMR** (250 MHz, d₆-DMSO):: δ(ppm) = 1.09 (3H, t, CH₃-ester, J = 7.0 Hz); 2.90 (2H, t, CH₂N, J = 7.0 Hz); 3.94 (3H, s, OCH₃); 4.01 (2H, q, CH₂-ester, J = 7.0 Hz); 4.38 (2H, t, CH₂C=O, J = 7.0 Hz); 6.48 (1H, d, H-C(5), J = 4.0 Hz); 7.48 (1H, d, H-C(6), J = 4.0 Hz).
   - **¹³C-NMR** (250MHz, d₆-DMSO):: δ(ppm) = 13.9 (CH₃-ester); 33.9 (CH₂C=O); 39.8 (CH₂-ester); 54.8 (OCH₃); 60.2 (CH₂N); 98.7 (C-5); 112.3 (C-4); 129.4 (C-6); 151.6 (C-7a); 152.8 (C-2); 160.6 (C-4a); 170.7 (C=O).
2. Step: 4-Chloro-7-(2-ethoxycarbonylethyl)-2-methoxy-7*H*-pyrrolo[2,3-d]pyrimidine (500 mg, 1.76 mmol) was dissolved in a mixture of 20 ml of EtOH/20 ml of 1 N NaOH and stirred for 30 min at room temperature. After dilution with water the reaction mixture was neutralized by addition of Amberlite IR-120 (H⁺-form, glass electrode). After filtration the ion exchange resin was filtered off and thoroughly washed with EtOH/H₂O (1:1, v/v), and the filtrate was evaporated to dryness. The residue was dissolved in a small amount of water, and 7-(2-carboxyethyl)-4-chloro-2-methoxy-7H-pyrrolo[2,3-d]pyrimidine was crystallized by addition of a few drops of glacial acetic acid. Colorless needles (368 mg, 82%);
   M.p. 140-142°C. TLC (silica gel, 0.25 M aq. LiCl): R_{f} 0.5.
   UV (MeOH): λₘₐₓ 227, 278, 297 nm (ε, 29.700, 4.500, 4.900).
   Anal. Calcd. for C₁₀H₁₀N₃O₃Cl (255.667): C, 46.98; H, 3.94; N, 16.44. Found: C, 46.87; H, 3.97; N, 16.34.
   - **¹H-NMR** (250 MHz, d₆-DMSO):: δ(ppm) = 2.82 (2H, t, CH₂N, J = 7.0 Hz); 3.96 (3H, s, OCH₃); 4.37 (2H, t, CH₂C=O, J = 7.0 Hz); 6.50 (1H, d, H-C(5), J = 4.0 Hz); 7.50 (1H, d, H-C(6), J = 4.0 Hz).

### Example 6

### Synthesis of 1-{2-[4-Chloro-7H-pyrrolo [2,3-d]pyrimidin-7-yl)ethyl]}-1'-[(2-diethoxy-phosphory)ethyl]-4-(2-pyridin-4-ylethyl)pyridinium Dibromide.

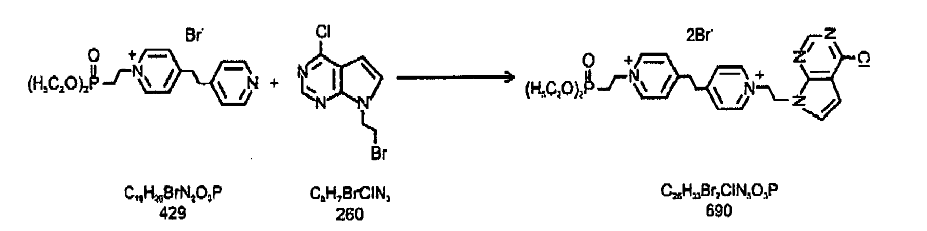

1-[2-(Diethoxyphosphoryl)ethyl]-4-(2-pyridine-4-yl-ethyl)pyridinium bromide (51 mg, 0.34 mmol and 182 mg (0.7 mmol) of 7-(2-bromoethyl)-4-chloro-*7H*-pyrrolo(2,3-*d*]pyrimidine were dissolved in 20 ml of MeCN and stirred at 70 °C for 7 days. After several hours the solution turned deep red, and after two days a deep red solid precipitated. After cooling the reaction mixture the deep red precipitate was filtered off, washed with ether and evaporated in vacuo to dryness. For further purification, the product was dissolved in 15 ml of H₂O. A dark purple precipitate was obtained. The precipitate was filtered off, and the residue was treated with 20 ml of H₂O several times in an ultrasound bath. The aqueous phase was concentrated to about half of the volume and 6 ml of a 10 % NH₄PF₆ solution was added. After drying the solid was dissolved in 10 ml of MeCN, and 8 ml of tetrabutylammoniumbromide (5 % in MeCN) solution was added dropwise. The deep-red precipitate was filtered off, and the solid was dissolved in MeOH and precipitated with ether overnight at 4 °C. After evaporating to dryness for 24 hours, 34 mg (0.05 mmol, 14 %) of a deep-red powder was obtained.
- **¹H-NMR** (250 MHz, d₆-DMSO):: δ(ppm) = 9.50 (b, 1 H), 8.78 (d, ³J[H,H] = 12.5 Hz, 2H), 8.61 (d, 3J[H,H] = 5.0 Hz, 2H), 7.81 (m, 4H), 7.47 (q, ³J [H,H] = 10.0 Hz, 1H), 6.34 (b, 1H), 5.41 (b, 2H), 4.86 (b, 2H), 3.80 (b, 4H), 3.42 (b, 2H), 3.22 (b, 2H), 2.61 (m, 4H), 1.05 (t, ³J[H,H] = 6.9 Hz, 6H).

### Example 7

### Synthesis of 1-{2-[4-chloro-7H-pyrrolo [2,3-d]pyrimidin-7-yl)ethyl]}4,4-bipyridinium bromide.

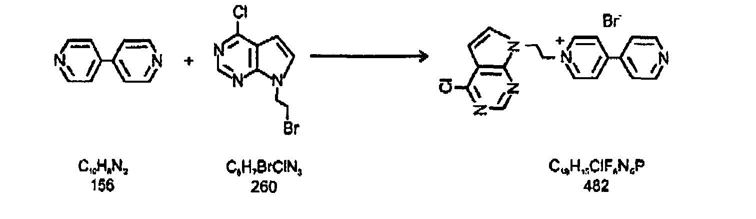

4,4'-Bipyridine (25 mg, 0.16 mmol) und 416 mg (1.6 mmol) of 7-(2-bromoethyl)-4-chloro-7*H*-pyrrolo[2,3-*d*]pyrimidine were dissolved in 20 ml of MeCN and stirred for 6 hours at 70 °C. An opaque yellowish solution was obtained. The mixture was concentrated completely, and the oily residue was dissolved in 10 ml of H₂O and treated in an ultrasound bath until a white powder appeared. This powder was filtered off and treated again three times with water in an ultrasound bath. The aqueous phases were concentrated and filtered with celite and concentrated to dryness. The yellowish solid was evaporated in vacuo to dryness. Yield: 34.5 mg (0.07 mmol, 44 %) of 1-{2-[4-chioro-*7H*-pyrrolo[2,3-*d*]pyrimidin-7-yl)ethyl]}-4.4'-bipyridinium bromide.
- **¹H-NMR** (250 MHz, d₆-DMSO):: δ(ppm) = 8.59 (d, ³J [H,H]= 5.0 Hz, 2H), 8.45 (t, ³J [H,H]= 10.0 Hz, 2H) 8.32 (d, ³J [H,H]= 2.5 Hz, 1 H), 8.02 (d, ³J [H,H]= 7.5 Hz, 2H) 7.58(t, ³J [H,H]= 10.0 Hz, 2H), 7.43 (t, ³J [H,H]= 5.0 Hz, 1H) 6.51 (d, ³J[H] = 2.5 Hz, 1H), 4.99 (t, ³J[N,H]=10.0 Hz, 2H), 4.81 (q, ³J[H,N]=15.0 Hz, 2H).
- **¹³C-NMR** (250 MHz, d₆-DMSO):: δ(ppm) = 154.9 (C7a-B), 152.4 (C4-B), 150,8 (C4'-Bypi), 150.3 (C2-B), 149.7 (CH-2',3'-Bipy), 145.4 (CH4',5' Bipy), 142.2 (C4-Bipy), 130.9 (C6-B), 126.3 (CH-2,3-Bipy), 122.4 (CH-4,5-Bipy), 117.6 (C4a-B), 99.8 (C5-B), 61.5 (C1-Ethyl-B), 45.6 (C2-Ethyl-Base),

### Example 8

### Synthesis of 1,1'-bis-{2-[4-chloro-7H-pyrrolo[2,3-d]pyrimidin-7-yl)ethyl]}-4,4'-bipyridinium dibromide.

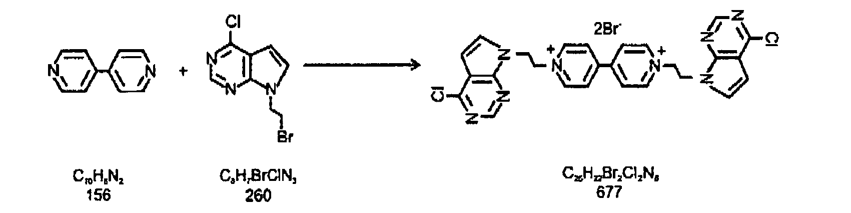

4,4'-Bipyridine (50 mg, 0.32 mmol) and 250 mg (0.96 mmol) of 7-(2-bromoethyl)-4-chloro-7*H*-pyrrolo[2,3-*d*]pylimidine were dissolved in 20 ml of MeCN and stirred at 70 °C for 24 hours. The product precipitated as light-brown solid. The cooled reaction mixture was filtered, washed three times with MeCN and ether and dried. The mother liquor was concentrated completely, and the residue was dried. The combined solids were suspended in 15 ml of H₂O and precipitated with 6 ml of a 10 % solution of NH₄PF₆. The precipitated brown solid was filtered, washed with H₂O and dried in vacuo.

For further purification, the product was dissolved in 10 ml of MeCN, and 10 ml of aqueous TBABr (5 %) were added dropwise. The precipitate was filtered off and washed thoroughly with MeCN (three times in an ultrasound bath). A white precipitate was obtained. The precipitate was filtered off, and the solvent was evaporated in vacuo. After drying in vacuo 48.3 mg (0.07 mmol, 22 %) of 1,1'-bis-{2-[4-chtoro-7*H*-pyrrolo[2,3-*d*]pyrimidin-7-yl)ethyl]}-4,4'-bipyridinium dibromide was obtained as a light-green powder.
- **¹H-NMR** (250 MHz, d₆-DMSO):: δ(ppm) = 9.15 (d, ³J [H,H] = 5.0 Hz, 4H), 8.51 (d, ³J [H,H] = 5.0 Hz, 4H) 8.32 (s, 2H), 7.75 (d, ³J [H,H] = 5.0 Hz, 2H), 6.72 (d, ³J [H,H] = 2.5 Hz, 2H), 5.16 (s, 4H), 4.98 (s, 4H).
- **¹³C-NMR** (250 MHz, d₆-DMSO):: δ(ppm) = 150.9 (C2,2'-B), 150.2 (C4,4',7a,7a'-B), 148.8 (C4,4'-Bipy), 131.4 (C6,6'-B), 126.9 (CH-2,3,5,6,2',3',5',6'-Bipy), 116.6 (C4a,4a'-B), 99.5 (C5,5'-B), 60.7 (C1-Ethyl-B), 45.3 (C2-Ethyl-Base),

### Example 9

### Synthesis of 1,1'-1"-[benzene-1,3,5-triyl-tris(methylene)]-tris-[1'-7-(2-ethyl)-4-chloro-7H-pyrrolo [2,3-d]pyrimidin-7-yl)ethyl]-4,4'-bipyridinium hexachloride

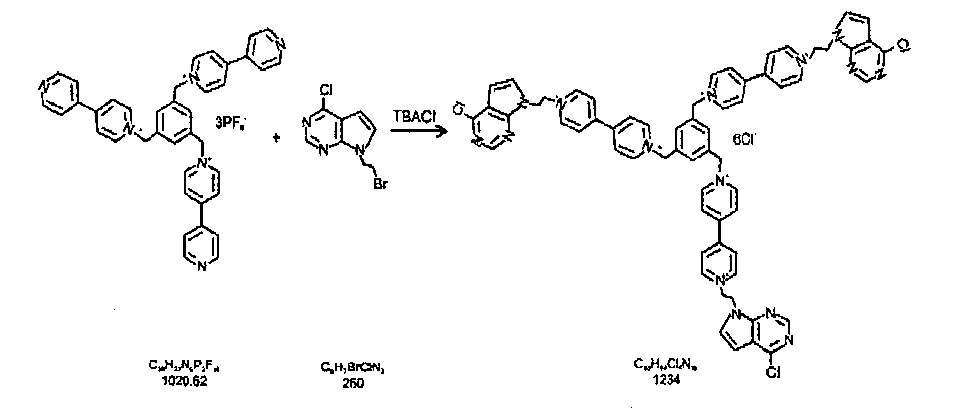

A solution of 81 mg (0.08 mmol) of 1,1',1"-[benzene-1,3,5-triyl-tris(methylene)tris](4-pyridin-4-ylpyridinium trihexafluorophosphate in 16 ml of MeCN were added in portions of 1 ml within four hours to a stirred solution of 104 mg (0.4 mmol) of 7-(2-bromoethyl)-4-chloro-7*H*-pyrrolo[2,3-*d*]pyrimidine in 10 ml of MeCN. The whole mixture was refluxed for three days at 70 °C.

The cooled reaction mixture was added dropwise to 15 ml of a stirred tetrabutylammoniumchloride solution (5 % in MeCN). The yellowish precipitate was filtered off and washed three times with 10 ml of MeCN and three times with 10 ml of CH₂Cl₂. After drying for 24 hours in vacuo 87 mg (0.067 mmol, 84 %) of 1,1',1"-[benzene-1,3,5-triyl-tris(methylene)]-tris-[1'-7-(2-ethyl)-4-chloro-7*H*-pyrrolo[2,3-*d*]pyrimidin-7-yl)ethyl]-4,4'-bipyridinium hexachloride was obtained as a brown powder.
- ¹H-NMR (250 MHz, D₂O):: δ(ppm) = 9.80 (d, ³J [H,H] = 7.5 Hz, 6H), 9.20 (d, ³J [H,H] = 7.5 Hz, 6H), 8.92 (s, 3H), 8,81 (d, ³J [H,H] = 7.5 Hz, 6H), 8,67 (d, ³J [H,H] = 7.5 Hz, 6H), 7.92 (d, ³J [H,H] = 2.5 Hz, 3H), 7.78 (s,3), 6.93 (d, ³J [H,H] = 7.5 Hz, 3H), 5.97 (s, 6H), 4.80 (t, ³J[N,H] = 5.0 Hz, 6H), 4.02 (t, ³J[H,H] =12.5 Hz, 6H).
- ¹³C-NMR (250 MHz, D₂O):: δ(ppm) = 153.3 (C2 x3-B) 152.1 (C7a x3-B), 149.5 (C4 x3-B), 145.1 (C4 x3 Bipy), 143.8 (C4' x3 Bipy), 134.8 (C-3,5,6-Benz.), (CH-2,4,6-Benz.), 126.9 (CH-Bipy) 126.6 (CH-Bipy) 110.3 (C 4a x3-B), 96,9 (C5 x3-B), 62.9 (C1-Benzyl.), 46.1 (C1- x3-Ethyl-B), 42.3 (C2 x3-Ethyl-Base)

### Example 10

### Synthesis of 1,1',1"=[benzene-1,3,5-triy-ltris(methylene)]tris-[1'-2-amino-7-(2-ethyl)-4-chloro-7H-pyrrolo [2,3-d]pyrimidin 7-yl)ethyl]-4,4'-bipyridinium hexabromide

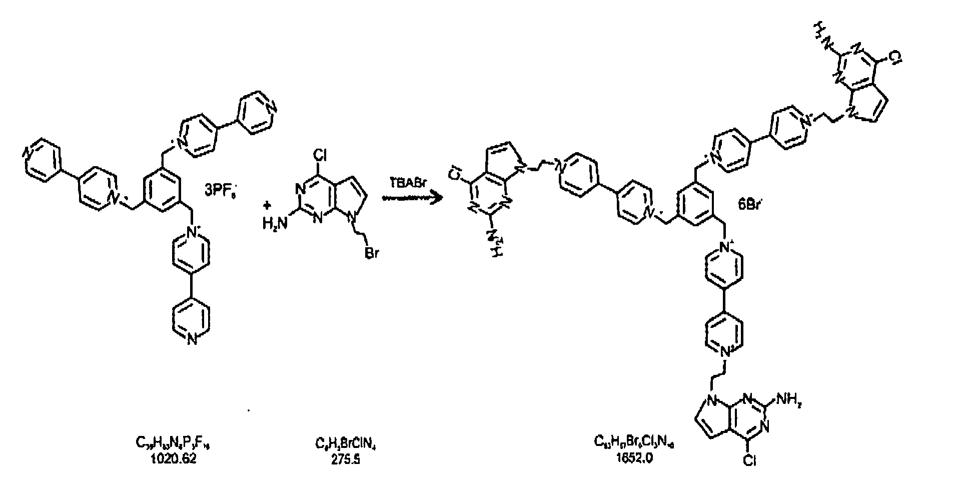

A solution of 81 mg (0.08 mmol) of 1,1',1"-[benzol-1,3,5-triyl-tris(methylene)tris](4-pyridine-4-ylpyridinium trihexafluorophosphate in 16 ml of MeCN were added dropwise in portions of 1 ml within four hours to a solution of 110 mg (0.4 mmol) of 2-amino-7-[bromomethyl]-4-chloro-7*H*-pyrrolo[2,3-*d*]pyrimidine in 16 ml of MeCN. The reaction mixture was refluxed for three hours at 70°C. The cooled reaction mixture was filtered and washed three times with MeCN and ether, respectively and dried. The mother liquor was evaporated and the residue was dried. The combined solids were re-suspended in 15 ml of H₂O and precipitated with 6 ml of a 10 % solution of NH₄PF₆. The residue was washed several times with water and dried in vacuo for 48 hours. Subsequently, anion exchange chromatography was applied with 103 mg of a Br-/PF-6 mixed salt of the crude product and 8 ml of a 5 % TBABr solution. 96 mg (0.058 mmol, 72 %) of 1,1',1"-[benzene-1,3,5-triyl-tds(methylene)]tris-[1'-2-amino-7(2-ethyl)-4-chloro-7*H*-pyrrolo[2,3-*d*]pyrimidin-7-yl)ethyl]-4,4'-bipyridinium hexabromide was obtained as a pink powder.
- ¹H-NMR (250 MHz, D₂O):: 6(ppm) = 9.11 (t, ³J [H,H] = 17.5 Hz, 3H), 8.93 (d, ³J [H,H] = 7.5 Hz, 8H), 8.59 (s, 6H), 8.31 (d, ³J [H,H] = 5.0 Hz, 8H), 7.91 (t, ³J [H,H] = 12.5 Hz, 8H), 7.66 (d, ³J [H,H] = 5.0 Hz, 3H), 7.41 (s, 3H), 5.86 (s, 6H), 4,78 (s, 6H), 3.22 (s, 6H)
- ¹³C-NMR (250 MHz, D₂O):: δ(ppm) = 157.1 (C2 x3-B) 153.9 (C7a x3-B), 149.5 (C4 x3-B), 144.9 (C4 x3 Bipy), 144.4 (C4' x3 Bipy), 136.1 (C-3,5,6-Benz.), 131,3 (CH-2,4,6-Benz.), 126.9 (CH-Bipy) 123.8 (CH-Bipy) 116.7 (C 4a x3-B), 97.8 (C5 x3-B), 62.6 (C1-Benzyl.), 58.9 (C1 x3-Ethyl-B), 45.1 (C2 x3-Ethyl-Base).

### Experiment 1

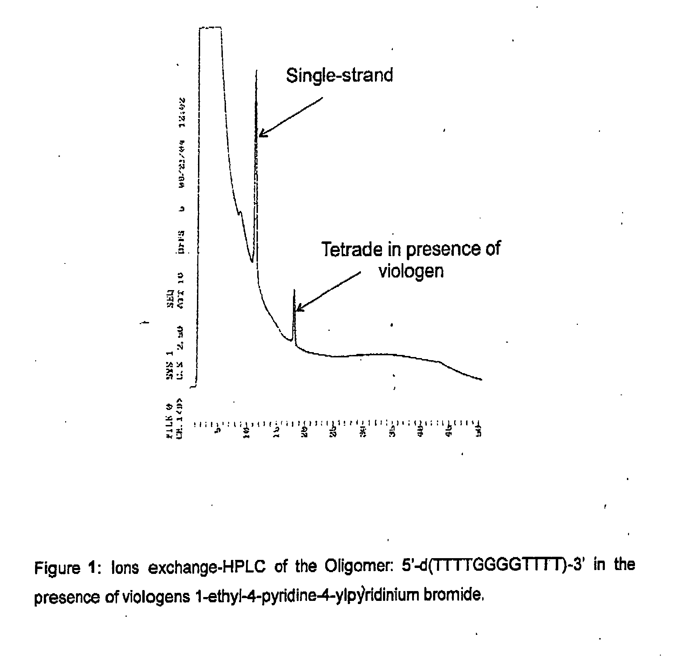

In a first experiment the interaction of a viologen molecule, 1-ethyl-4-pyridine-4-ylpyridinium bromide and the oligonucleotide 5'-d(TTT GGGG TTT) was examined. This oligomer forms tetrades in the presence of K⁺ ions but not in the presence of Li⁺-ions. Tetrade and single strand molecules which are present in an equilibrium may be detected simultaneously by ion-exchange HPLC. When performing ion-exchange chromatography of said oligomer in 1 M LiCl, 25 mmol TRIS-HCl, 1 mmol EDTA, pH 8, in the presence of the viologen 1-ethyl-4-pryidine-4-ylpyridinium bromide, but in the absence of K⁺-ions, the HPLC profile shows the presence of tetrades beside the presence of a single strand (see figure 1).

### Experiment 2

In a second experiment dendrimers of the order zero with pending bases at the periphery are synthesized. In the case that the dendritic branches are synthesized first and, thereafter, said branches are arranged at the core moiety; said type of synthesis is called convergent synthesis (C. Hawker, J.M.J. Frechet, "Journal of the Chemical Society-Chemical Communications", 1990, p. 1010; T.M. Miller, T.X. Neenan, "Chemistry of Materials", 1990, 2, p. 346. The synthesis shown in scheme 16 for the 0^{th} order dendrimers relates to a divergent synthesis starting from the core to the periphery (N. Ardoin, D. A. Struc, Bull. Soc. Chim. France, 1995, 132, 875-909; G. R. Newkome, C. N. Moorfield, F. Vögtle, "Dendritic Molecules: Concepts, Syntheses, Perspectives", VCH, Weinheim, 1996). The inner trifunctional core 3 was synthesized either starting from a mesitylen derivative with N-bromsuccinimide (NBS), Route I.a. (F. Vögtle, M. Zuber, R.G. Lichtenthaler, Chem. Ber, 1973, 106, 717-718) or starting from commercially available trimesine acid (Route I.b): (W.P. Cochrane, P.L. Pauson, T.S. Stevens, J. Chem. Soc., Perkin Transaction, 1968, 630-632, A. J. Y. Lan, R. O. Heuckeroth, J. Amer. Chem. Soc. 1987, 109, 2738-2745). 1,3,5-Tris(brommethyl)benzene 3 is suitable as a core moiety since the 4,4'-bipyridinium units are particularly suitable for alkylation with benzylic halogens with a Menschutkin-reaction for alkylation (D.B. Amabilino, R.P. Ashton, M. Belohradsky, F. M. Raymo, J. F. Stoddart, J. Chem. Soc., Chem. Commun. 1995, 751-753). The nucleophilic N-atoms at the periphery in compound 4 are reactive positions which may be reactive with alkyl-, benzyl- or phenyl halogens. The periphery of the dendrimers consist of substituted pyrrolo[2,3-*d*]pyrimidine units, which were reacted with 1,2-dibromoethane in advance (Route I.c). Finally, the 0^{th} order dendrimer 7 was obtained by alkylation of the precursor of 4 with the alkylated pyrrolo[2,3-*d*]pyrimidine 6 in MeCN with a yield of over 50 %.

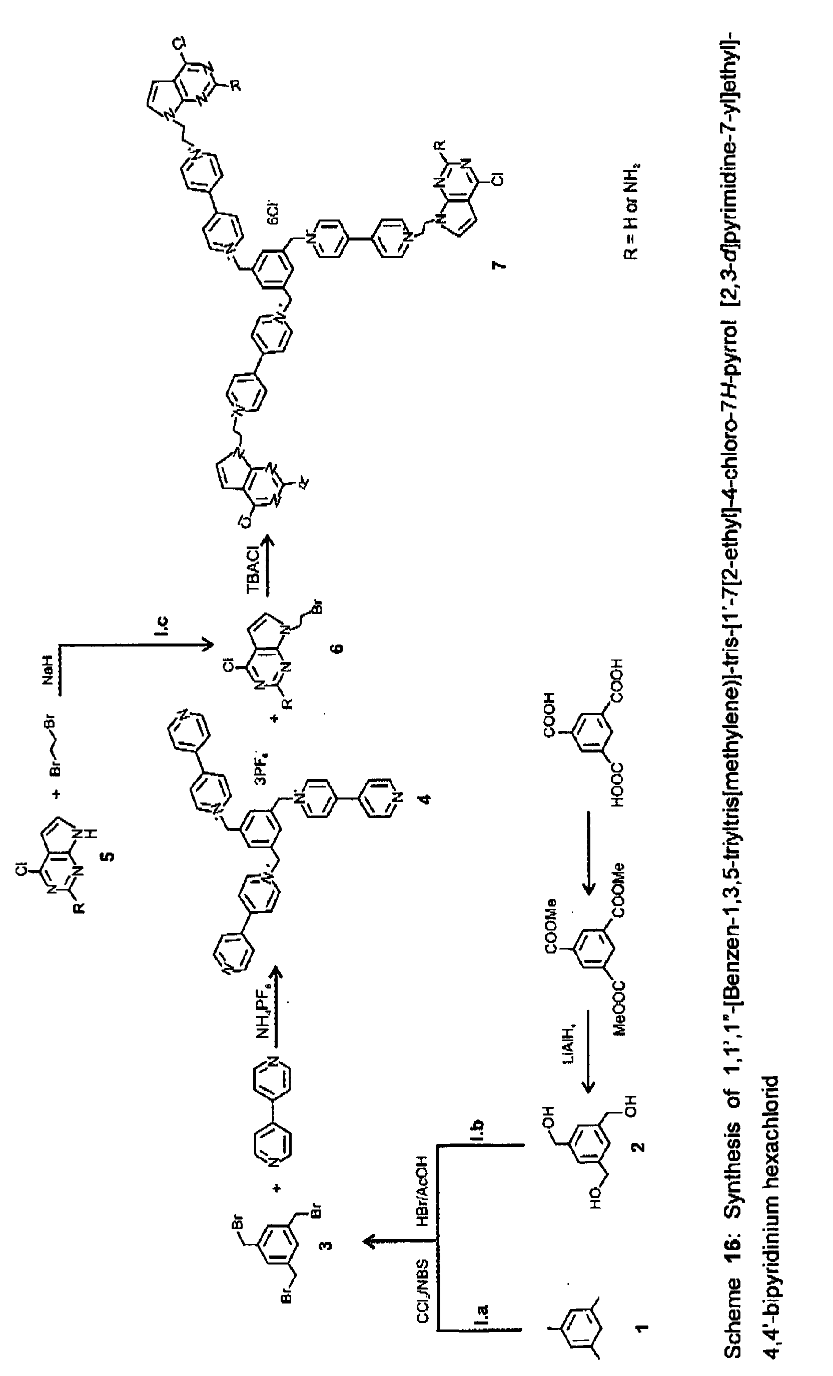

Due to their redox properties the viologen units may be examined by spectroelectrochemistry since they change their colour depending on their redox status. The monomeric viologens have typically absorption maxima in the range between 300 to 750 nm. Benzyl-substituted viologens have typically absorption maxima at 401 nm (ε = 28900 M⁻¹cm⁻¹), 608 nm (ε = 11700 M⁻¹cm⁻¹), phenyl-substituted viologens show typically an absorption maxima at 446 nm (ε = 29000 M⁻¹ cm⁻¹), 714 nm (ε = 15600 M⁻¹ cm⁻¹). In case of pimere the maximum is shifted to about 370 nm and 550 nm. In addition, in the long-wavelength range at about 900 nm a new band appears which seems to be due to charge-transfer-complexes of said compounds. (see e.g.: Susanne Heinen, University of Osnabrück, Thesis 1999).

### Experiment 3

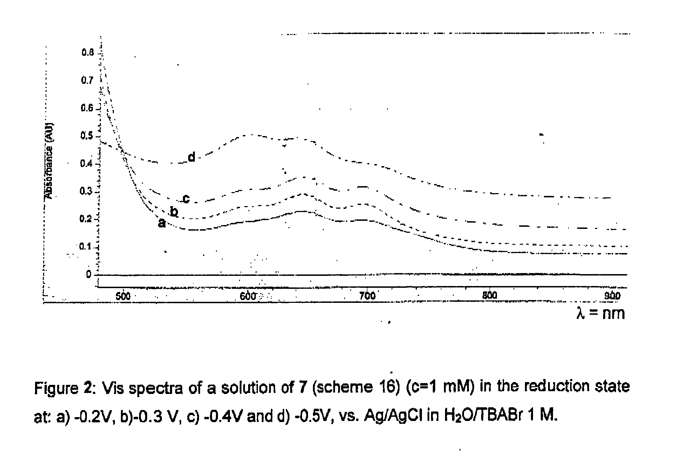

In a third experiment, the new hybrid compound 7 (scheme 16) having a viologen skeleton was examined with the help of a spectroelectrochemical cell in solution consisted of a divided cell with 1 cm cuvette in the working electrode compartiment.The working electrode was 0,073 g of graphitized carbon felt GFA-5 of approximately 0.073 m² BET area from SGL carbon, the electrochemically active area of which is not known. The absorption spectra of said compound 7 were determined at different potentials. The potential was decreased step-wise by about 100 mV, and the equilibrium adjustment was awaited before recording the spectrum. The spectro-electrochemical measurements on compound 7 (c = 1 mM) were performed in H₂O with 1 M TBABr (tetrabutylammonium bromide) as lead salt at room temperature in a three-electrode system *versus* Ag/AgCl. For each measurement the solution was purged with argon to cast out oxygen. The measurement was started at 0 V and was continued to -0.600 V. The absorption maxima was at 595 nm (εₘₐₓ = 50400 M⁻¹cm⁻¹), 630 nm (εₘₐₓ = 49200 M⁻¹cm⁻¹) and at 700 nm (εₘₐₓ = 40200 M⁻¹cm⁻¹) and increase when decreasing the potential to about - 0.500 V (see figure 2).

The colour of the solution was deep green in the reduced state and yellowish in the oxidized state.

### Experiment 4

In a fourth experiment, compound 7 was absorbed on a TiO₂-electrode and examined.

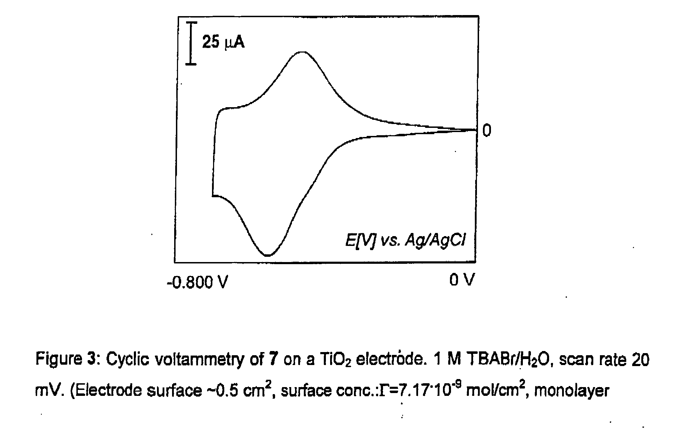

Colloidal TiO₂ was coated on 10 mm x 10 mm FTO glass electrodes (F-doped SnO₂ 15 Ω/cm²) paste from *LOF Industries,* using the *doctor-blade* method to yield ~5 µm thick films (Literature: F. Campus., et. al. Electrochromic devices based on surface-modified nano-crystalline TiO2 thin-film electrodes, Solar Energy Materials and Solar Cells. 1999, 56(3-4), 281-297.) The TiO₂ electrode was exposed to a 1 mM solution in H₂O of the pyrrolo[2,3-*d*]pyrimidine derivative 7 equipped with viologen units for 1h at r.t. and rinsed with water. The spectro-electrochemical and CV-measurements of 7-on-TiO₂ were performed in a three electrode arrangement (reference Ag/AgCl) in a 1 M TBABr in H₂O. Cyclic voltammetry of the 7-on-TiO₂ electrode shows reversible waves (figure 3).

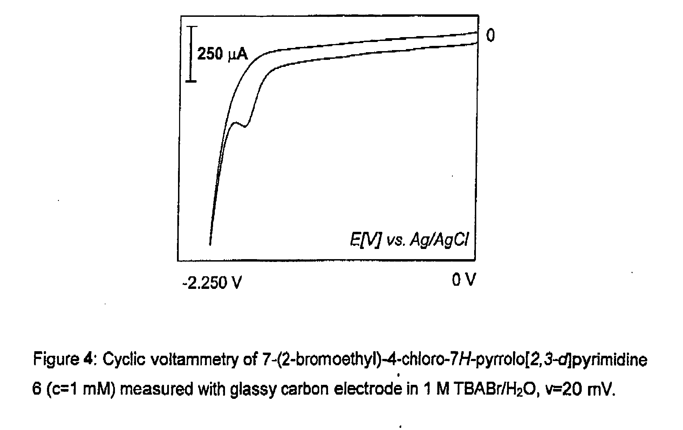

The reduction potential is observed at -0.551 V and is typical for viologen units because the redox potential of pyrrolo[2,3-*d*]pyrimidine units 6 (Schema 16) is highly negativ (-1.784 V measured with a glassy carbon electrode, figure 4).

The modified electrode of 7 (monolayer) shows an intense red coloration brought about by a new pyrrolo[2,3-*d*]pyrimidine bypyridinium salt combined with an amplification caused by the monocrystalline TiO₂ electrode and is, therefore, interesting for electrochromic application. The coloration of a transparent TiO₂ electrode can be amplified by a cross-linking reaction.

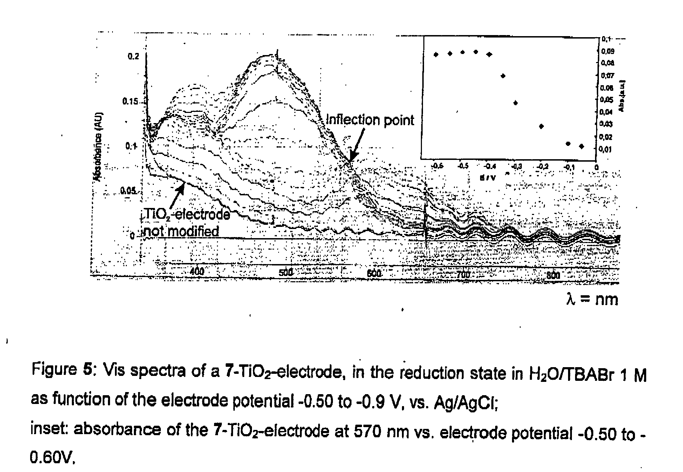

Spectroelectrochemistry of 7-on-TiO₂ is possible because of its fast response (figure 5). The spectral changes as a function of the applied potential is as follows: between 540 and 700 nm the absorbance of the 7-on-TiO₂ electrode increases with decreasing potential (until -0.6 V); upon a further decrease of the potential (-0.6 to -1 V) the absorbance between 380 and 570 nm increases significantly.

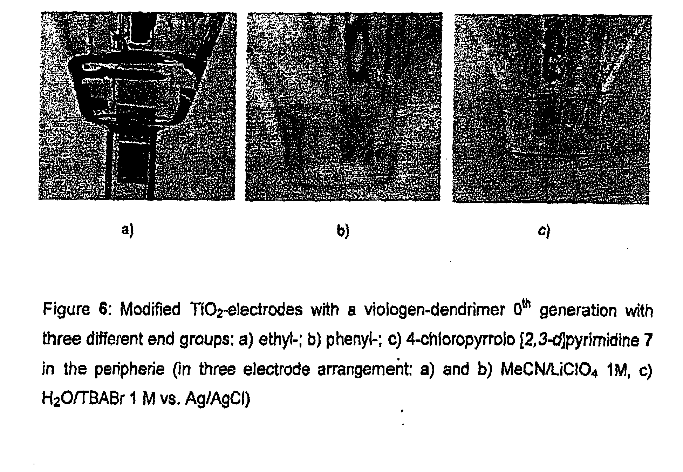

Figure 6 shows the effect of a heterocyclic base as an end group and in a dendrimer (0^{th} generation) with viologen as redox units as well as the coloration of such electrodes compared with well-known viologen dendrimers.

In figure 6a, the electrode has a blue colour, typically for absorbed alkyl viologen with discontinuous Π-conjugation. In figure 6b, the electrode has a green colour, typically for absorbed phenylviologen where the Π-electron system of the bipyridine is extended to the aryl residues. In figure 6c the electrode has a red colour of the new compound having an extended Π-electron system. The new compound 7 is a combination of the blue viologen and a pyrrolo[2,3-*d*]pyrimidine derivative, wherein the Π-conjugation is bridged via an ethylene residue. Although this results in a dramatic extension of the Π-electron system, the pyrrolo[2,3-*d*]pyrimidine has a strong influence on the absorption spectrum as well as on the redox potential (-0.550 mV) in comparison to dendrimers of 0^{th} order with ethyl residues at the periphery as shown in figure 6a.

## Claims

1. A hybrid compound having i) a moiety of an N-heterocyclic nucleobase or a derivative thereof, ii) a redox active moiety and iii) a spacer moiety linking the nucleobase moiety with the redox active moiety.

2. The hybrid compound according to claim 1, wherein the redox active moiety is selected from the group consisting of a 4,4'-bipyridine, a 2,2'-dipyridine, a 1,2-di(4-pyridyl)ethane, a 2,9 diazaperylene, a 2,7-diazapyrene, or derivatives thereof which may be substituted.

3. The hybrid compound according to claim 2 wherein the redox active moiety is a 1,1'-disubstituted 4,4'-dipyridine.

4. The hybrid compound according to any one of claims 1 to 3 wherein the spacer is selected from the group consisting of a C₁-C₁₂ alkyl-, alkenyl-, alkynyl-, or aryl group or a (CH₂CH₂-O)ₙ-group with n being an integer of 1 to 100.

5. The hybrid compound according to claim 4 wherein the spacer is a C₁-C₂ alkyl- or alkenyl-group.

6. The hybrid compound according to any one of the preceding claims which has at least two nucleobase moieties as defined above which may be identical or different from each other.

7. The hybrid compound according to any one of claims 1 to 6 containing further a core moiety connecting at least two hybrid units of i) the nucleobase moiety, ii) the redox active moiety and iii) a spacer moiety linking the nucleobase moiety with the redox active moiety.

8. The hybrid compound according to any one of the preceding claims wherein the spacer moiety enables the formation of a conjugated Π-system between the nucleobase moiety and the redox active moiety.

9. A method for the production of hybrid compounds having (i) a moiety of an N-heterocyclic nucleobase or a derivative thereof, ii) a redox active moiety and iii) a spacer moiety comprising the step of (a) reacting said N-heterocylic nucleobase or derivative thereof with a spacer molecule whereby said spacer molecule has at least two reactive groups to obtain an intermediate compound consisting of said N-heterocylic nucleobase or derivative thereof and a spacer moiety whereby said spacer moiety remains one reactive group.

10. A method according to claim 9 further comprising the step of (b) reacting the intermediate compound consisting of said nucleobase moiety and a spacer moiety wherein the spacer moiety has one reactive group with a redox active moiety to obtain compounds according to any one claims 1 to 8.

11. An electrochromic material comprising hybrid compounds as defined in any one of claims 1 to 8.

12. A polymer comprising hybrid compounds having a moiety of i) a nucleobase or a derivative thereof, ii) a redox moiety and iii) a spacer moiety linking the nucleobase moiety with the redox moiety as defined in any one of claims 1 to 8.

13. A dendrimer composed of hybrid compounds according to any one of claims 1 to 8.

14. The dendrimer according to claim 13, wherein the core molecule(s) are linked with the compounds having i) a nucleobase moiety, ii) a redox moiety and iii) a spacer moiety linking the nucleobase moiety with the redox moiety via a connector group.

15. The dendrimer according to claim 14, wherein the connector group is selected from the group consisting of C₁-C₁₂ alkyl-, alkenyl-, alkynyl- or aryl-group or (CH₂CH₂-O)n-group with n being an integer of 1 to 100.

16. An electrochromic device, in particular an electrode, comprising a layer of electrochromic material comprising the compounds as defined in any one of claims 1 to 8.

17. The electrochromic device according to claim 16 further comprising TiO₂ nanocrystals sintered on Indium tin oxide layers.

18. The electrochromic device according to claim 16 or 17 wherein the spacer is a C₁ to C₂ alkyl- or alkenyl-group.

19. The hybrid molecules according to any one of claims 1 to 8 wherein the moiety of a nucleobase or a derivative thereof is a moiety of a nucleoside or nucleotide or a derivative thereof.

20. A nucleic acid molecule comprising a hybrid molecule according to claim 19.

21. A biosensor containing a hybrid molecule according to any one of claims 1 to 8, 19 or 20 or electrochromic material according to claim 11 or dendrimers or polymers according to claims 12 to 15.
